(19) Europäisches Patentamt
European Patent Office
Office européen des brevets

(11) EP 4 725 933 A1

# (12) EUROPEAN PATENT APPLICATION

published in accordance with Art. 153(4) EPC

(43) Date of publication:
**15.04.2026 Bulletin 2026/16**

(21) Application number: **24823226.6**

(22) Date of filing: **28.05.2024**

(51) International Patent Classification (IPC):
***C07C 35/29*** *(2006.01)* ***C07C 35/31*** *(2006.01)*
***C07F 7/12*** *(2006.01)* ***C08K 3/00*** *(2018.01)*
***C08K 3/08*** *(2006.01)* ***C08K 5/05*** *(2006.01)*
***C08K 5/13*** *(2006.01)* ***C08L 83/05*** *(2006.01)*
***C08L 83/07*** *(2006.01)*

(52) Cooperative Patent Classification (CPC):
**C07C 35/29; C07C 35/31; C07F 7/12; C08K 3/00; C08K 3/08; C08K 5/05; C08K 5/13; C08L 83/04**

(86) International application number:
**PCT/JP2024/019556**

(87) International publication number:
**WO 2024/257606 (19.12.2024 Gazette 2024/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **12.06.2023 JP 2023096071**

(71) Applicant: **SHIN-ETSU CHEMICAL CO., LTD.**
**Tokyo 100-0005 (JP)**

(72) Inventors:
- **KITAZAWA, Keita**
  **Annaka-shi, Gunma 379-0224 (JP)**
- **IKENO, Masayuki**
  **Annaka-shi, Gunma 379-0224 (JP)**

(74) Representative: **Schicker, Silvia**
**Wuesthoff & Wuesthoff**
**Patentanwälte und Rechtsanwalt PartG mbB**
**Schweigerstraße 2**
**81541 München (DE)**

## (54) BICYCLIC ACETYLENE ALCOHOL COMPOUND, ADDITION-CURABLE SILICONE COMPOSITION, AND THERMALLY CONDUCTIVE ADDITION-CURABLE SILICONE COMPOSITION

(57) The present invention is a bicyclic acetylene alcohol compound, wherein the carbon atom on which the hydroxy group of the compound is substituted is a tertiary carbon atom bonded to an acetylene group, and an $\alpha$-carbon of the compound is a bridgehead carbon atom, or does not have a hydrogen atom on the $\alpha$-carbon, or satisfies both of these conditions. This can provide: a bicyclic acetylene alcohol compound having difficulty in undergoing an isomerization reaction to an $\alpha,\beta$-unsaturated ketone; an addition-curable silicone composition containing the compound; and a thermally conductive addition-curable silicone composition containing the compound.

EP 4 725 933 A1

## Description

### TECHNICAL FIELD

The present invention relates to a bicyclic acetylene alcohol compound, an addition-curable silicone composition, and a thermally conductive addition-curable silicone composition.

### BACKGROUND ART

Addition-curable silicone composition cures to form a silicone gel, a silicone rubber, a hard coat film, etc., which are excellent in electrical properties, cold resistance, heat resistance, and chemical stability. Therefore, they can be widely used as a sealing material, a filler, and a coating material for electrical and electronic parts and semiconductor elements, and a protective insulating and coating agent for optical semiconductor, etc. Further, by blending various inorganic fillers, it is possible to increase strength of a composition and impart heat resistance. Furthermore, it is also used as a heat dissipation material and a conductive material for a semiconductor element and an electronic component, such as an LED substrate.

To initiate a curing reaction (hydrosilylation) of an addition-curable silicone composition at the desired timing, controlling a pot life and a shelf life is crucial, and several methods for the control have been proposed for this purpose. For example, a microcapsule structure embedding a hydrosilylation-reaction catalyst within a polymer compound has been proposed (e.g., Patent Documents 1 and 2). However, the hydrosilylation-reaction catalyst embedded within the microcapsule structure exhibits a slow diffusion rate into the composition, resulting the problem that the addition-curing reaction is difficult to initiate.

As another means, an addition-curing reaction-controlling agent selected from an acetylene compound, a nitrogen compound, an organophosphorus compound, an oxime compound, an organochlorine compound, etc., may be blended (Patent Document 3). These addition-curing reaction-controlling agents can control a pot life and a shelf life by incorporating an appropriate amount, enabling a curing reaction of the addition-curable silicone composition to be initiated at the desired timing. Acetylene alcohols are frequently used because they are low-odor and have a low environmental impact.

As described above, the acetylene alcohols are useful as an addition-curing reaction-controlling agent. However, it is known that in the presence of acid, they undergo a reaction that isomerize them into an $\alpha,\beta$-unsaturated ketone (Meyer-Schuster rearrangement, Rupe rearrangement) (Non-Patent Document 1). Ability of the isomerized $\alpha$, $\beta$-unsaturated ketone to control an addition-curing reaction is significantly reduced. Consequently, there was a problem where the pot life or the shelf life of an addition-curable silicone composition shortened markedly.

### CITATION LIST

#### PATENT LITERATURE

Patent Document 1: JPH11-236508A
Patent Document 2: JP2014-024986A
Patent Document 3: JPH04-046962A

#### NON PATENT LITERATURE

Non Patent Document 1: J. Org. Chem. 1977, 42, 3403

### SUMMARY OF INVENTION

#### TECHNICAL PROBLEM

Accordingly, the present invention has been made in view of the above-described problem, and an object of the present invention is to provide: a bicyclic acetylene alcohol compound having difficulty in undergoing an isomerization reaction to an $\alpha,\beta$-unsaturated ketone; an addition-curable silicone composition containing the compound; and a thermally conductive addition-curable silicone composition containing the compound.

SOLUTION TO PROBLEM

To solve the problems above, the present invention provides a bicyclic acetylene alcohol compound, wherein the carbon atom on which the hydroxy group of the compound is substituted is a tertiary carbon atom bonded to an acetylene group, and an $\alpha$-carbon of the compound is a bridgehead carbon atom, or does not have a hydrogen atom on the $\alpha$-carbon, or satisfies both of these conditions. Here, the term "$\alpha$-carbon" refers to the two carbon atoms other than an acetylene group adjacent to the carbon atom on which the hydroxy group of the compound is substituted.

By using such a bicyclic acetylene alcohol compound, the isomerization reaction to an $\alpha,\beta$-unsaturated ketone is difficult to occur, making it difficult to shorten a pot life or a shelf life when incorporated as an addition-curing reaction-controlling agent in the addition-curable silicone composition or the thermally conductive addition-curable silicone composition.

Further, in the present invention, the minimum number of carbon atoms constituting a ring is preferably 7 or fewer.

By using such a bicyclic acetylene alcohol compound, the isomerization reaction to an $\alpha,\beta$-unsaturated ketone is further difficult to occur, making it difficult to shorten a pot life or a shelf life when incorporated as an addition-curing reaction-controlling agent in the addition-curable silicone composition or the thermally conductive addition-curable silicone composition.

Further, the present invention provides an addition-curable silicone composition comprising an effective amount of the bicyclic acetylene alcohol compound described above as an addition-curing reaction-controlling agent.

Such an addition-curable silicone composition makes an addition-curable silicone composition whose pot life or shelf life are difficult to shorten.

Further, the present invention provides a thermally conductive addition-curable silicone composition comprising:

(A) organopolysiloxane having at least one aliphatic unsaturated hydrocarbon group per molecule and a kinematic viscosity at 25°C of 60 to 100,000 $mm^2/s$: an amount of constituting 1.5 to 89 mass% relative to the entire composition;
(B) at least one thermally conductive filler selected from the group consisting of a metal, a metal oxide, a metal hydroxide, a metal nitride, a metal carbide, and a carbon allotrope: an amount of 10 to 98 mass% relative to the entire composition;
(C) organohydrogenpolysiloxane having two or more hydrogen atoms bonded to a silicon atom per molecule: an effective amount sufficient for the composition to form a cured product;
(D) a platinum group metal catalyst: an effective amount; and
(E) the bicyclic acetylene alcohol compound according described above: an effective amount.

Such a thermally conductive addition-curable silicone composition makes an addition-curable silicone composition whose pot life or shelf life are difficult to shorten, which has excellent heat-dissipating properties.

In this event, the component (B) is preferably silver powder.

Such a thermally conductive addition-curable silicone composition has further excellent heat dissipation properties.

Further, in the present invention, the thermally conductive addition-curable silicone composition preferably additionally comprises

(F) a phenol compound represented by the following general formula (1A): an amount of 0.01 to 10 mass% relative to the entire composition,

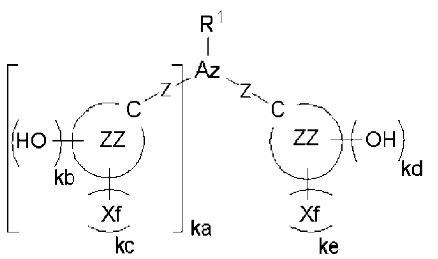
(1A)

wherein $R^1$ represents a hydrogen atom, a halogen atom, a cyano group, or a hydroxy group; Az represents a linear, branched, or cyclic (ka+2)-valent hydrocarbon group having 1 to 20 carbon atoms or a linear, branched, or cyclic (ka+2)-valent fluorinated hydrocarbon group having 1 to 20 carbon atoms, wherein $-CH_2-$ constituting the hydrocarbon group is optionally substituted with -O-, -C(=O)-, or $-Si(R^2R^3)-$; $R^2$ and $R^3$ represent a linear, branched, or cyclic alkyl group having 1 to 6 carbon atoms or a phenyl group, wherein $-CH_2-$ constituting the alkyl group is optionally substituted with -O-, -C(=O)-, or $-Si(CH_3)_2-$; Xf each independently represents a hydrogen atom, a halogen atom, a

linear, branched, or cyclic monovalent hydrocarbon group having 1 to 10 carbon atoms and optionally being substituted with a fluorine atom, an alkoxy group having 1 to 10 carbon atoms and optionally being substituted with a fluorine atom, or an electron-withdrawing group; Z represents a single bond or an oxygen atom; rings ZZ each independently represent an aromatic monocyclic or polycyclic ring having 3 to 20 carbon atoms, wherein a carbon atom of the ring ZZ is optionally substituted with a nitrogen atom, an oxygen atom, or a sulfur atom; ka represents an integer of 0 to 2; kb and kd represent 1 or 2; and kc and ke represent an integer of 0 to 2.

Such a thermally conductive addition-curable silicone composition has excellent heat-dissipating properties more reliably.

In this event, the component (F) is preferably a phenol compound represented by the following general formula (1B),

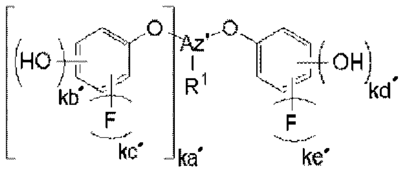 (1B)

wherein Az' represents a linear, branched, or cyclic (ka'+2)-valent hydrocarbon group having 1 to 19 carbon atoms or a linear, branched, or cyclic (ka'+2)-valent fluorinated hydrocarbon group having 1 to 19 carbon atoms, and $-CH_2-$ constituting the hydrocarbon group is optionally substituted with -O-, -C(=O)-, or - Si ($R^2R^3$) -; ka' represents 0 or 1; kb', kc', kd' , and ke' represent 1 or 2; and $R^1$, $R^2$, and $R^3$ are same as defined above.

Such a thermally conductive addition-curable silicone composition exhibits excellent heat-dissipating properties further reliably.

ADVANTAGEOUS EFFECTS OF INVENTION

As described above, the inventive bicyclic acetylene alcohol compound is a tertiary acetylenic alcohol compound, and an α-carbon is a bridgehead carbon atom, or does not have a hydrogen atom on the α-carbon, or satisfies both of these conditions, so that an isomerization reaction to an α, β-unsaturated ketone is difficult to occur. Consequently, it becomes difficult to shorten a pot life or a shelf life when incorporated as an addition-curing reaction-controlling agent in the addition-curable silicone composition or the thermally conductive addition-curable silicone composition.

DESCRIPTION OF EMBODIMENTS

As described above, it has been desired to develop a bicyclic acetylene alcohol compound that is difficult to undergo an isomerization reaction to an α,β-unsaturated ketone.

As a result of their diligent study of the problems above, the inventors found that a tertiary acetylenic alcohol compound, whose an α-carbon was a bridgehead carbon atom, or did not have a hydrogen atom on the α-carbon, or satisfied both of these conditions, could solve the problem above, and have completed the present invention.

That is, the present invention is a bicyclic acetylene alcohol compound, wherein the carbon atom on which the hydroxy group of the compound is substituted is a tertiary carbon atom bonded to an acetylene group, and an α-carbon of the compound is a bridgehead carbon atom, or does not have a hydrogen atom on the α-carbon, or satisfies both of these conditions.

Hereinafter, the present invention will be specifically described, but the present invention is not limited thereto. In the following description, some structures represented by the chemical formula may have an asymmetric carbon, which allows for the existence of enantiomers and diastereomers; in such cases, a single formula represents all of these isomers. One of these isomers may be used, or they may be used in a mixture.

[Bicyclic Acetylene Alcohol Compound]

The present invention is a bicyclic acetylene alcohol compound, wherein the carbon atom on which the hydroxy group of the compound is substituted is a tertiary carbon atom bonded to an acetylene group, and an α-carbon of the compound is a bridgehead carbon atom, or does not have a hydrogen atom on the α-carbon, or satisfies both of these conditions. Such a compound can be suitably used as an addition-curing reaction-controlling agent for an addition-curable silicone composition or a thermally conductive addition-curable silicone composition.

In the present invention, the term "α-carbon" refers to the two carbon atoms other than an acetylene group

adjacent to the carbon atom on which the hydroxy group of the compound is substituted.

This structure makes it difficult for an isomerization reaction to α, β-unsaturated ketone to occur, thereby making it difficult to reduce ability to control an addition-curing reaction. Consequently, the pot life and the shelf life of the addition-curable silicone composition and the thermally conductive addition-curable silicone composition have difficulty in being shortened.

Specific examples of the bicyclic acetylene alcohol compound include the following.

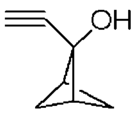
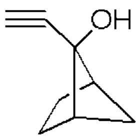
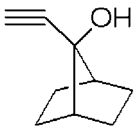
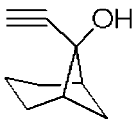
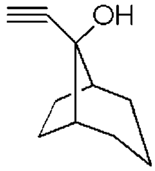
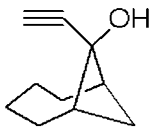
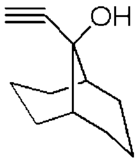
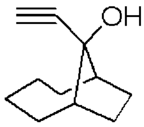
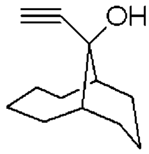
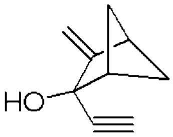
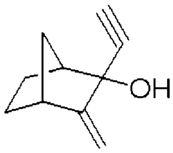
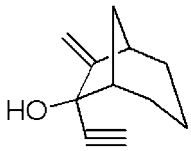
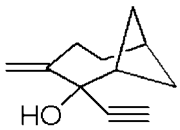
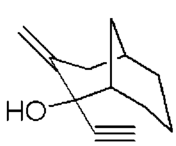
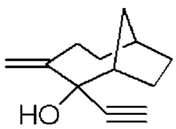
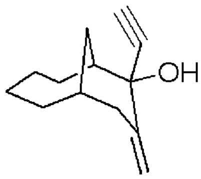
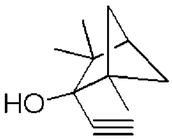
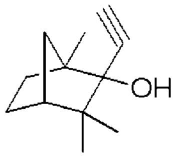
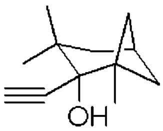
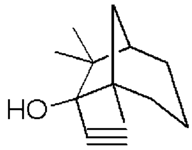
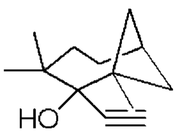
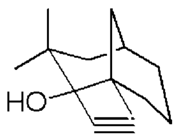
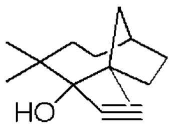
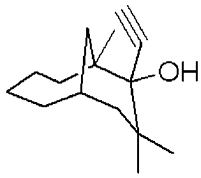

The synthesis method for the inventive bicyclic acetylene alcohol compound is not particularly limited, the optimal method for the synthesis may be selected according to the structure. For example, it can be obtained by reacting corresponding bicyclic ketone compound with an ethynyl magnesium halide and followed by treating with acid. If necessary, it can be purified by a conventional method such as distillation, recrystallization, or chromatography.

Furthermore, the inventive bicyclic acetylene alcohol compound has the minimum number of carbon atoms constituting a ring is preferably 7 or fewer. The lower limit for the minimum number of carbon atoms constituting a ring may be, for example, three.

A cyclic alkene having a ring with seven or fewer members and a trans double bond cannot exist stably. Therefore, a bicyclic acetylene alcohol compound, which has the minimum number of carbon atoms constituting a ring of seven or fewer, has difficulty in undergoing an isomerization reaction to α, β-unsaturated ketone. Consequently, the ability

to control an addition-curing reaction is difficult to reduce, making it difficult to shorten the pot life and the shelf life of an addition-curable silicone composition and a thermally conductive addition-curable silicone composition.

[Addition-Curable Silicone Composition]

The inventive addition-curable silicone composition contains an effective amount of the bicyclic acetylene alcohol compound described above as an addition-curing reaction-controlling agent.

Molecular structure and blending amount of organopolysiloxanes or organohydrogenpolysiloxanes having an aliphatic unsaturated hydrocarbon group within the addition-curable silicone composition, presence or absence, a type, and a blending amount of a filler, a type and a blending amount of a curing catalyst, and presence or absence of other components are not particularly limited. However, the inventive bicyclic acetylene alcohol compound is particularly effective from the viewpoint of being capable of extending the pot life and the shelf life of compositions containing an acidic component.

[Thermally Conductive Addition-Curable Silicone Composition]

The inventive thermally conductive addition-curable silicone composition contains a bicyclic acetylene alcohol compound described above as the component (E), and the components (A) to (D) described below, and in addition to these, the component (F) and other components as necessary. Each component is will be described in detail below.

<u>Component (A)</u>

The component (A) is organopolysiloxane having at least one, preferably 1 to 100, and more preferably 2 to 50 aliphatic unsaturated hydrocarbon groups per molecule and a kinematic viscosity at 25°C of 60 to 100,000 $mm^2/s$.

The aliphatic unsaturated hydrocarbon group is preferably a monovalent hydrocarbon group having an aliphatic unsaturated bond and having 2 to 8 carbon atoms, more preferably 2 to 6 carbon atoms, and further preferably an alkenyl group. Examples thereof include alkenyl groups, such as a vinyl group, an allyl group, a propenyl group, an isopropenyl group, a butenyl group, a hexenyl group, a cyclohexenyl group, and an octenyl group. It is particularly preferably a vinyl group. The aliphatic unsaturated hydrocarbon group may be bonded to any of silicon atoms at a terminal or in middle of the molecule chain, or may be bonded to both of them.

An organic group, bonded to a silicon atom of the organopolysiloxane, other than the aliphatic unsaturated hydrocarbon group is an unsubstituted or substituted monovalent hydrocarbon group having 1 to 18, preferably 1 to 10, and more preferably 1 to 8 carbon atoms. Examples thereof include: alkyl groups, such as a methyl group, an ethyl group, a propyl group, an isopropyl group, a butyl group, an isobutyl group, a tert-butyl group, a pentyl group, a neopentyl group, a hexyl group, a cyclohexyl group, an octyl group, a nonyl group, and a decyl group; aryl groups, such as a phenyl group, a tolyl group, a xylyl group, and a naphthyl group; aralkyl groups, such as a benzyl group, a phenylethyl group, and a phenylpropyl group; or groups in which a part or all of hydrogen atoms in these groups are substituted with a halogen atom such as fluorine, bromine, and chlorine, and a cyano group, which are, for example, a chloromethyl group, a chloropropyl group, a bromoethyl group, a trifluoropropyl group, and a cyanoethyl group. It is particularly preferably a methyl group or a trifluoropropyl group.

The organopolysiloxane has a kinematic viscosity at 25°C of 60 to 100,000 $mm^2/s$, and preferably 100 to 30,000 $mm^2/s$. When the kinematic viscosity is 60 $mm^2/s$ or more, physical properties of the thermally conductive addition-curable silicone composition is excellent. When the kinematic viscosity is 100,000 $mm^2/s$ or less, spreadability of the thermally conductive addition-curable silicone composition is excellent.

In the present invention, the kinematic viscosity is a value measured at 25°C with an Ubbelohde-type Ostwald viscometer (the same applies hereinafter).

A molecular structure of the organopolysiloxane is not particularly limited as long as the organopolysiloxane has the above properties, and examples thereof include a linear structure, a branched structure, a partially branched structure, or a linear structure having a cyclic structure. The organopolysiloxane particularly preferably has a linear structure in which the main chain is composed of repetition of a diorganosiloxane unit and both terminals of the molecule chain are blocked with a triorganosiloxy group. The organopolysiloxane having the linear structure may partially have a branched structure or a cyclic structure.

A blending amount of the component (A) is preferably 1.5 to 89 mass%, more preferably 1.7 to 50 mass%, and further preferably 2 to 20 mass% relative to the entire composition. When the blending amount of the component (A) is 89 mass% or less, thermal conductivity is excellent, and when the blending amount is 1.5 mass% or more, the viscosity of the composition does not unnecessarily increase, resulting excellent operability.

One of the organopolysiloxane may be used, or two or more kinds thereof may be used in combination.

Component (B)

The component (B) is one or more thermally conductive fillers selected from the group consisting of a metal, a metal oxide, a metal hydroxide, a metal nitride, a metal carbide, and a carbon allotrope. Examples include aluminum, silver, copper, metallic silicon, zinc oxide, magnesium oxide, aluminum oxide (alumina), silicon dioxide, cerium oxide, iron oxide, aluminum hydroxide, cerium hydroxide, aluminum nitride, boron nitride, silicon carbide, diamond, graphite, carbon nanotubes, and graphene. However, because of its superior thermal conductivity, it is preferably silver, particularly preferably silver powder. One kind of these may be used, or two or more kinds thereof may be used in combination, with their proportions being arbitrary and not specifically limited.
The average particle size of the component (B) is preferably within the range of 0.01 to 300 μm, more preferably within the range of 0.1 to 100 μm, and further preferably within the range of 1 to 50 μm. This is because a size of 0.01 μm or greater ensures that the viscosity of the resulting composition does not become excessively high while providing excellent spreadability, and a size of 300 μm or less ensures that the resulting composition is uniform. The average particle size can be determined, for example, as the volume-based average (or the median diameter) in particle size distribution measurements according to a laser diffraction method.
Additionally, one kind of the component (B) may be used, or two or more kinds thereof may be used in combination, with their proportions being arbitrary and not specifically limited.
The shape of the component (B) is not particularly limited and may be flake-shaped, spherical, granular, irregular, dendritic, needle-shaped, etc.
The blending amount of the component (B) is preferably 10 to 98 mass% relative to the entire composition, more preferably 40 to 96 mass%, and further preferably 70 to 95 mass%. When the blending amount of the component (B) is 98 mass% or less, the composition exhibits excellent spreadability, and when it is 10 mass% or more, it exhibits excellent thermal conductivity.

Component (C)

The component (C) is an organohydrogenpolysiloxane having two or more hydrogen atoms bonded to a silicon atom per molecule, that is, an organohydrogenpolysiloxane having two or more hydrogen atoms bonded to a silicon atom (SiH group) within a single molecule, particularly preferably 2 to 100, and more preferably 2 to 50. The organohydrogenpolysiloxane may be any type capable of undergoing an addition reaction between its SiH group and an aliphatic unsaturated hydrocarbon group present in the aforementioned component (A), in the presence of the platinum group metal catalyst as the component (D) described below, thus to form a crosslinked structure.
The organohydrogenpolysiloxane is not particularly limited in its molecular structure as long as it has the properties above. Examples include a linear structure, a branched structure, a cyclic structure, a partially branched structure, or linear structures with a cyclic segments. Preferably, it is a linear structure or a cyclic structure.
The organohydrogenpolysiloxane has a kinematic viscosity at 25°C of preferably 1 to 1,000 $mm^2/s$, more preferably 10 to 300 $mm^2/s$. When the dynamic viscosity is 1 $mm^2/s$ or higher, the physical properties of the thermally conductive addition-curable silicone composition become excellent. When the dynamic viscosity is 1,000 $mm^2/s$ or lower, the spreadability of the thermally conductive addition-curable silicone composition becomes favorable.
The organic groups bonded to a silicon atom of the organohydrogenpolysiloxane include unsubstituted or substituted monovalent hydrocarbon groups other than aliphatic unsaturated hydrocarbon groups. Specifically, they are unsubstituted or substituted monovalent hydrocarbon groups having 1 to 12 carbon atoms, preferably 1 to 10 carbon atoms. Examples include alkyl groups, such as a methyl group, an ethyl group, a propyl group, a butyl group, a hexyl group, and a dodecyl group; aryl groups, such as a phenyl group; aralkyl groups, such as a 2-phenylethyl group and a 2-phenylpropyl group; and groups in which part or all of the hydrogen atoms of these groups above are substituted with a halogen atom, such as a fluorine atom, a bromine atom, or a chlorine atom, a cyano group, an epoxy ringcontaining organic group (a glycidyl groups or a glycidoxy-substituted alkyl groups), or the like. Examples thereof include a chloromethyl group, a chloropropyl group, a bromoethyl group, a trifluoropropyl group, a cyanoethyl group, a 2-glycidoxyethyl group, a 3-glycidoxypropyl group, and a 4-glycidoxybutyl group. Among these, a methyl group and a trifluoropropyl group are preferable.
One kind of the organohydrogenpolysiloxane may be used, or two or more kinds thereof may be used in mixture.
The blending amount of organohydrogenpolysiloxane as the component (C) is an effective amount sufficient for the composition to form a cured product, preferably an amount where the number of SiH groups in the component (C) is 0.5 to 10 relative to the total number of aliphatic unsaturated hydrocarbon groups in the component (A), more preferably 0.7 to 7.5, and further preferably 1.0 to 5.0. When the blending amount of the component (C) is at or above the above lower limit, the addition reaction proceeds sufficiently, resulting in adequate crosslinking. Furthermore, when the blending amount is at or below the above upper limit, the crosslinked structure becomes uniform, leading to good storage stability of the composition.

Component (D)

The component (D) is a platinum group metal catalyst, and functions to promote the addition-curing reaction of the aforementioned components. A conventionally known platinum group metal catalyst used for the addition-curing reaction may be used as the platinum group metal catalyst. Examples include platinum-based, palladium-based, and rhodium-based catalysts, but platinum or platinum compounds, which are relatively readily available, are preferable. Examples include elemental platinum, platinum black, chloroplatinic acid, platinum-olefin complexes, platinum-alcohol complexes, and platinum coordination compounds. One kind of platinum group metal catalysts may be used, or two or more kinds thereof may be used in combination.

The blending amount of the component (D) may be the effective amount as a catalyst, i.e., the effective amount necessary to promote the addition-curing reaction and cure the inventive thermally conductive addition-curable silicone composition. Preferably, the blending amount is 0.1 to 500 ppm, more preferably 0.5 to 300 ppm, and further preferably 1 to 200 ppm, in terms of mass of platinum group metal atoms, relative to the entire composition. When the blending amount of the component (D) is at or above the above lower limit, a catalytic effect is obtained. When it is at or below the above upper limit, the catalytic effect is sufficient and economical.

Component (E)

The inventive thermally conductive addition-curing silicone composition incorporates the component (E) bicyclic acetylene alcohol compound described above as an addition-curing reaction control agent.

The blending amount of the component (E) may be an effective amount, and is preferably 0.05 to 10 parts by mass relative to 100 parts by mass of the component (A), more preferably 0.07 to 5 parts by mass, and further preferably 0.1 to 2 parts by mass. When the blending amount of the component (E) is 0.05 parts by mass or more, a sufficiently desired shelf life and pot life are obtained. Furthermore, when the amount is 10 parts by mass or less, the silicone composition exhibits sufficient curability.

One kind of the component (E) bicyclic acetylene alcohol compound may be used, or two or more kinds thereof may be used in combination.

Additionally, the component (E) may be diluted with organopolysiloxane, toluene, or the like in order to improve its dispersibility in the thermally conductive addition-curable silicone composition.

The inventive thermally conductive addition-curable silicone composition may optionally contain the following additional components as needed, in addition to the above components.

Component (F)

The component (F) is a phenol compound and is blended as an additive to the thermally conductive addition-curable silicone composition. The phenol compound is preferably a phenol compound represented by the following general formula (1A). In the following description, some structures represented by the chemical formula may have an asymmetric carbon, which allows for the existence of enantiomers and diastereomers; in such cases, a single formula represents all of these isomers. One of these isomers may be used, or they may be used in a mixture.

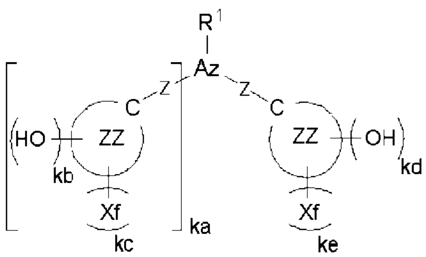

(1A)

In the formula (1A), $R^1$ represents a hydrogen atom, a halogen atom, a cyano group, or a hydroxy group; Az represents a linear, branched, or cyclic (ka+2)-valent hydrocarbon group having 1 to 20 carbon atoms or a linear, branched, or cyclic (ka+2)-valent fluorinated hydrocarbon group having 1 to 20 carbon atoms, wherein $-CH_2-$ constituting the hydrocarbon group is optionally substituted with -O-, -C(=O)-, or $-Si(R^2R^3)-$; $R^2$ and $R^3$ represent a linear, branched, or cyclic alkyl group having 1 to 6 carbon atoms or a phenyl group, wherein $-CH_2-$ constituting the alkyl group is optionally substituted with -O-, -C(=O)-, or $-Si(CH_3)_2-$; Xf each independently represents a hydrogen atom, a halogen atom, a linear, branched, or cyclic monovalent hydrocarbon group having 1 to 10 carbon atoms and optionally being substituted with a fluorine atom, an alkoxy group having 1 to 10 carbon atoms and optionally being substituted with a fluorine atom, or an electron-withdrawing group; Z represents a single bond or an oxygen atom; rings ZZ each independently represent an

aromatic monocyclic or polycyclic ring having 3 to 20 carbon atoms, wherein a carbon atom of the ring ZZ is optionally substituted with a nitrogen atom, an oxygen atom, or a sulfur atom; ka represents an integer of 0 to 2; kb and kd represent 1 or 2; and kc and ke represent an integer of 0 to 2.

Specific examples of the linear, branched, or cyclic (ka+2)-valent hydrocarbon group Az having 1 to 20 carbon atoms include the following.

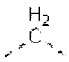
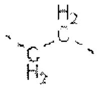
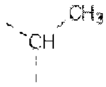
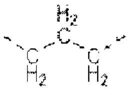
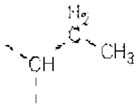
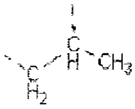
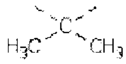

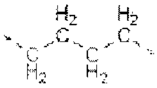
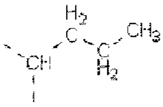
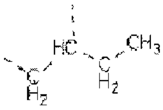
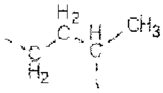
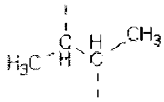

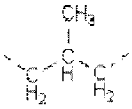
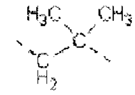
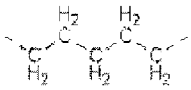
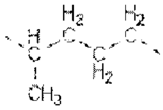
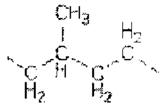

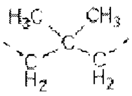
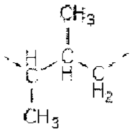
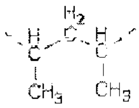
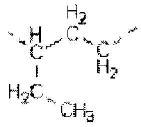
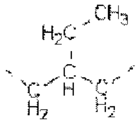
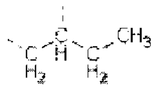

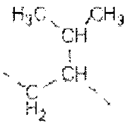
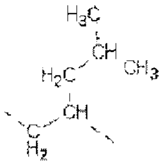
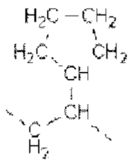
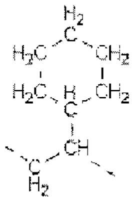
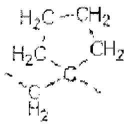
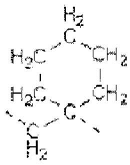

In the formulae, a dotted line represents an attachment point.

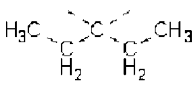
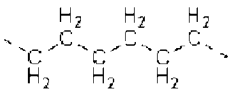
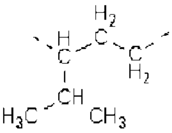
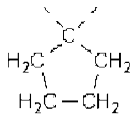
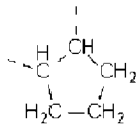

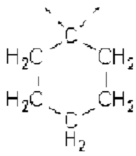
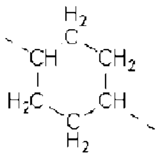
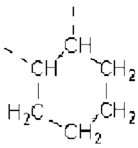
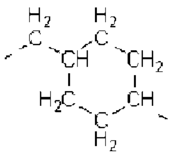
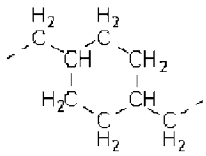

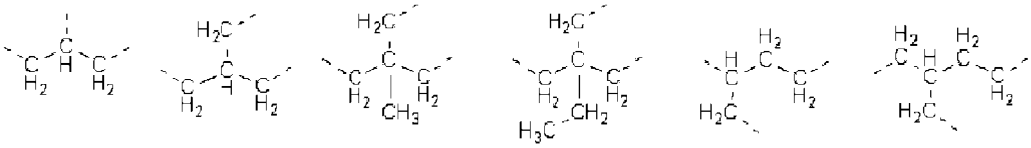

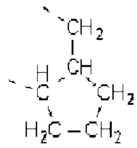 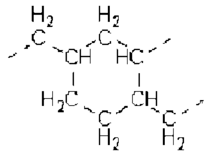 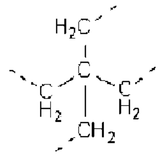 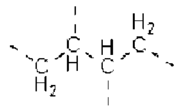 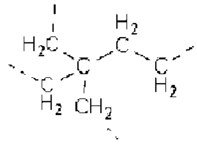

In the formulae, a dotted line represents an attachment point.

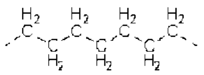 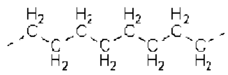 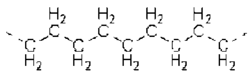 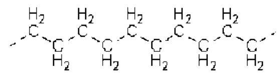

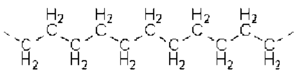 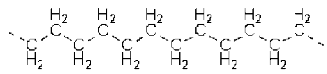 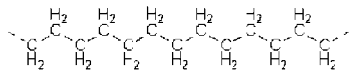

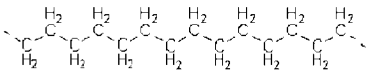 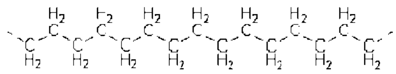

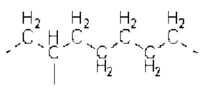 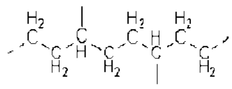 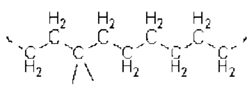 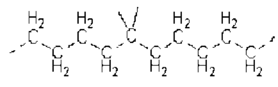

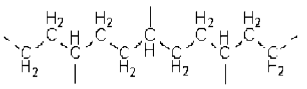 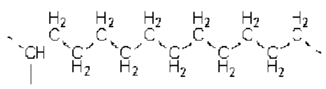 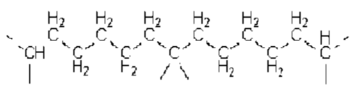

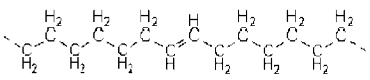 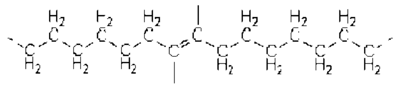

In the formulae, a dotted line represents an attachment point.

Specific examples of the linear, branched, or cyclic (ka+2)-valent fluorinated hydrocarbon group Az having 1 to 20 carbon atoms include groups in which part or all of the hydrogen atoms in the hydrocarbon group above are substituted with a fluorine atom. In addition, $-CH_2-$ constituting the hydrocarbon group may be substituted with -O-, -C(=O)-, or $-Si(R^2R^3)-$. The number of carbon atoms in Az is preferably 2 to 18, more preferably 3 to 16, and further preferably 4 to 14. Part of $-CH_2-$ constituting the hydrocarbon group are preferably substituted with $-Si(R^2R^3)-$ or $-Si(R^2R^3)-O-$.

Specific examples of the linear, branched, or cyclic alkyl groups $R^2$ and $R^3$ having 1 to 6 carbon atoms include a methyl group, an ethyl group, a propyl group, an isopropyl group, an n-butyl group, a sec-butyl group, a tert-butyl group, an n-pentyl group, an n-hexyl group, a cyclopropyl group, a cyclobutyl group, a cyclopentyl group, and a cyclohexyl group.

Examples of the halogen atom as Xf include a fluorine atom, a chlorine atom, a bromine atom, and an iodine atom.

Specific examples of the linear, branched, or cyclic monovalent hydrocarbon group, as Xf, having 1 to 10 carbon atoms and optionally substituted with a fluorine atom include: alkyl groups, such as a methyl group, an ethyl group, a propyl group, an isopropyl group, an n-butyl group, a sec-butyl group, a tert-butyl group, a cyclopropyl group, and a cyclobutyl group; a trifluoromethyl group; and a 2,2,2-trifluoroethyl group.

Specific examples of the alkoxy group, as Xf, having 1 to 10 carbon atoms and optionally substituted with a fluorine atom include a methoxy group, an ethoxy group, a propoxy group, an isopropoxy group, an n-butoxy group, a sec-butoxy group, a tert-butoxy group, a cyclopropoxy group, a trifluoromethoxy group, and a 2,2,2-trifluoroethoxy group.

Specific examples of the electron withdrawing group as Xf include a carbonyl group, an alkoxycarbonyl group, a cyano group, a nitro group, a sulfo group, a formyl group, a sulfonate ester group, an amide group, and -O-C(=O)-G- (G represents a sulfur atom or NH).

Specific examples of the aromatic monocyclic or polycyclic ring ZZ having 3 to 20 carbon atoms include the following. As described below, the ring ZZ may further have a substituent.

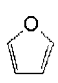 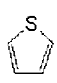 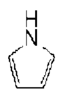 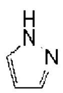 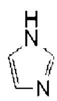 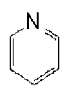 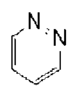 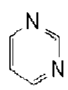 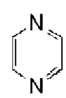 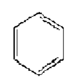

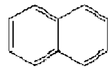 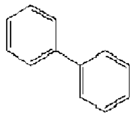 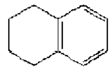 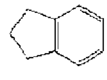 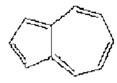 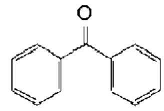

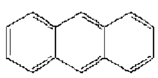 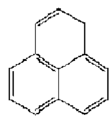 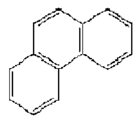 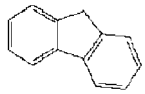 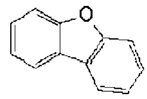 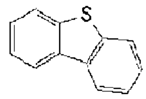

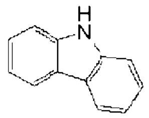 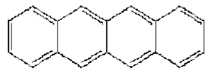 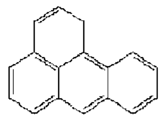 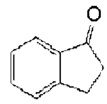 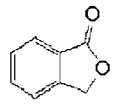

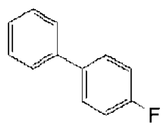 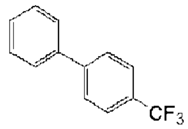 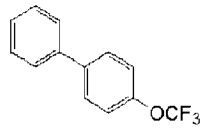 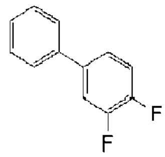 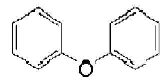

The component (F) above is particularly preferably a phenol compound represented by the following general formula (1B).

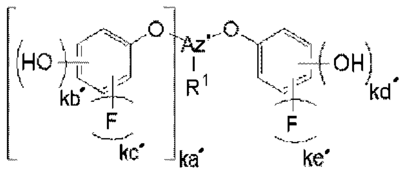 (1B)

In the general formula (1B), Az' represents a linear, branched, or cyclic (ka'+2)-valent hydrocarbon group having 1 to 19 carbon atoms or a linear, branched, or cyclic (ka'+2)-valent fluorinated hydrocarbon group having 1 to 19 carbon atoms, and $-CH_2-$ constituting the hydrocarbon group is optionally substituted with -O-, - C(=O)-, or $-Si(R^2R^3)-$; ka' represents 0 or 1; kb', kc', kd', and ke' represent 1 or 2; and $R^1$, $R^2$, and $R^3$ are same as defined above.

Specific examples of phenol compounds represented by the above general formulae (1A) and (1B) include the following.

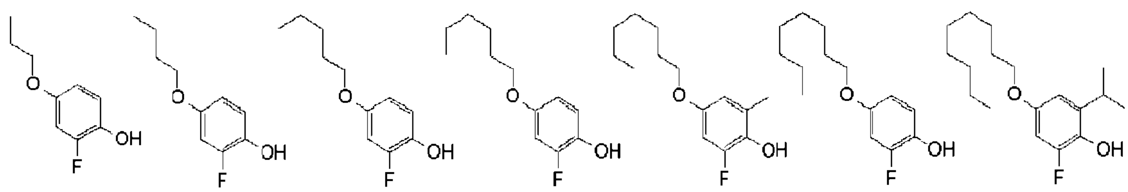

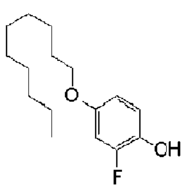 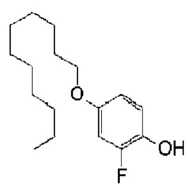 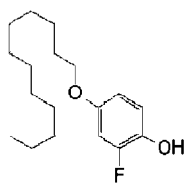 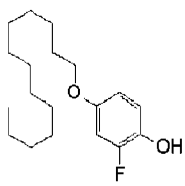 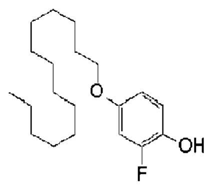

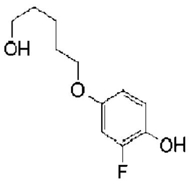 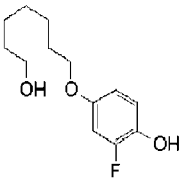 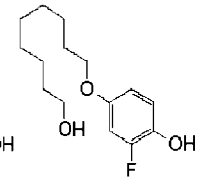 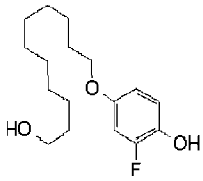 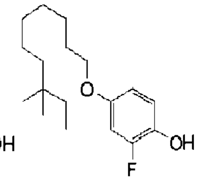 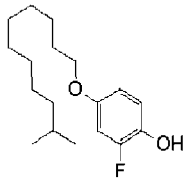

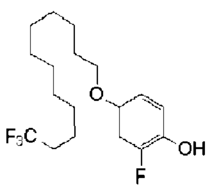 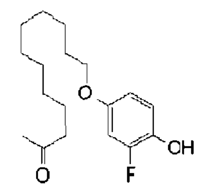 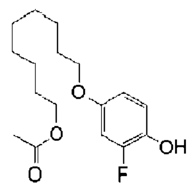 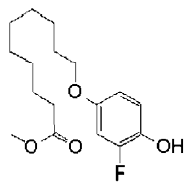 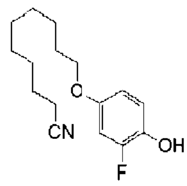

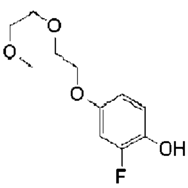 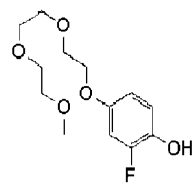 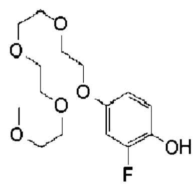 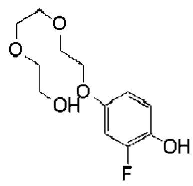 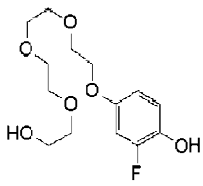

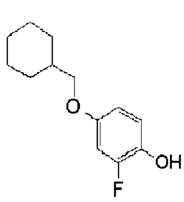 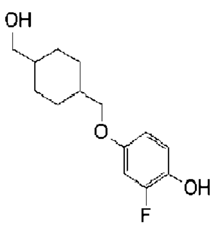 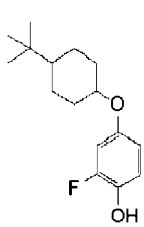 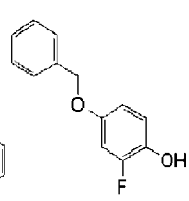 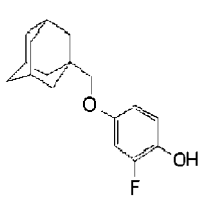 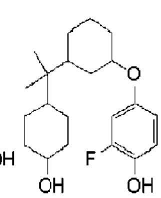

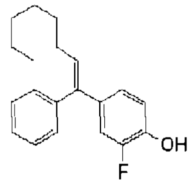

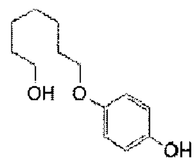 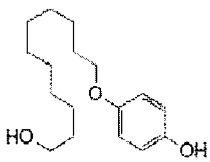 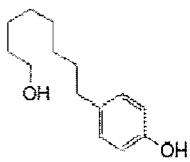 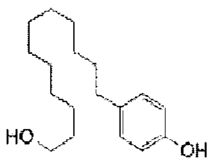 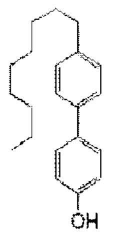 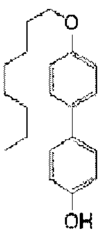

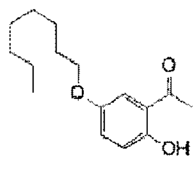 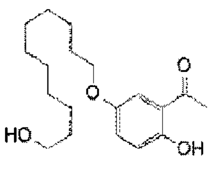 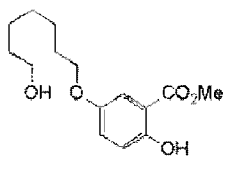 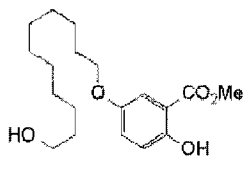 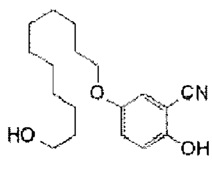

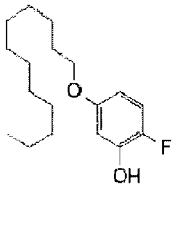 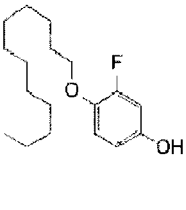 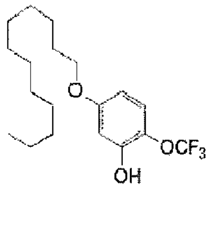 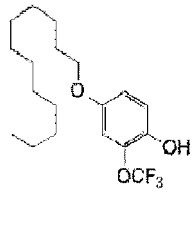 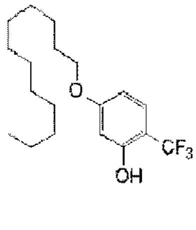

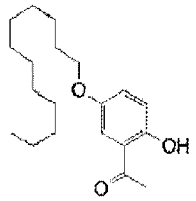 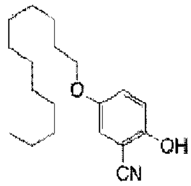

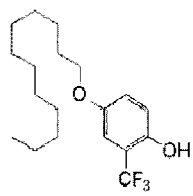 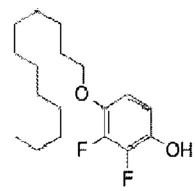 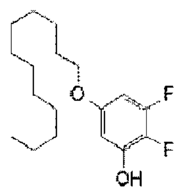 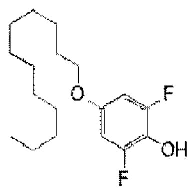 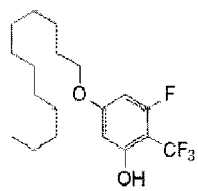

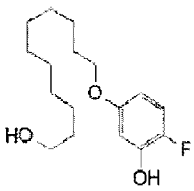 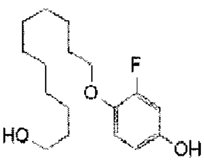 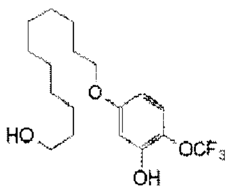 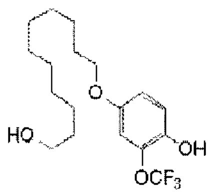 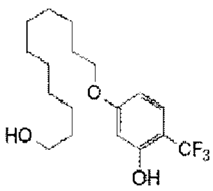

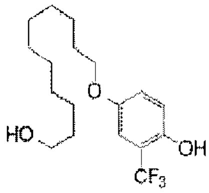 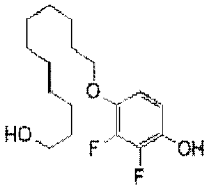 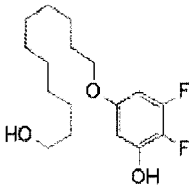 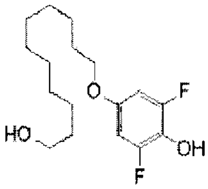 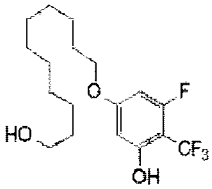

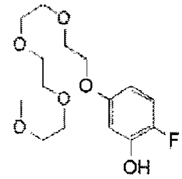 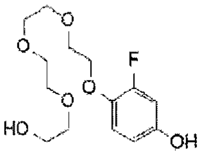 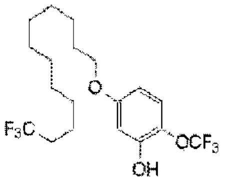 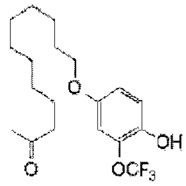 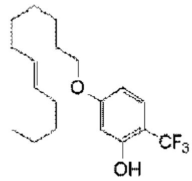

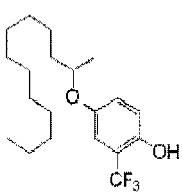 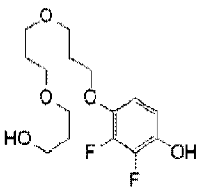 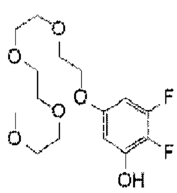 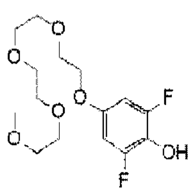 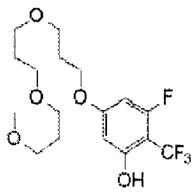

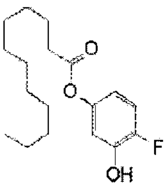 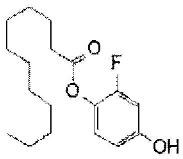 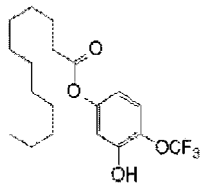 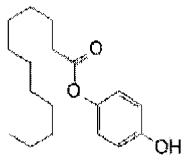 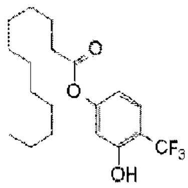

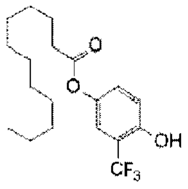
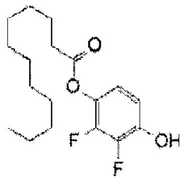
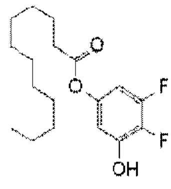
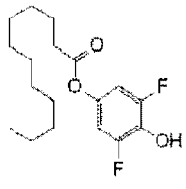
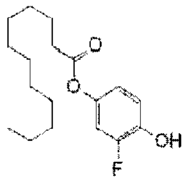

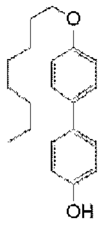
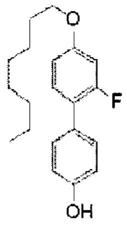
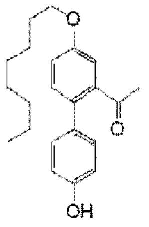
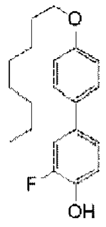
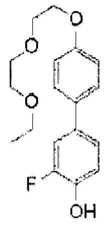
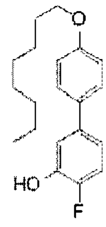
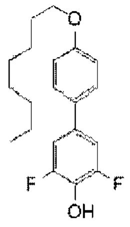

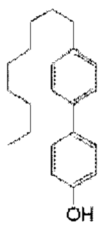
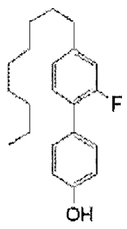
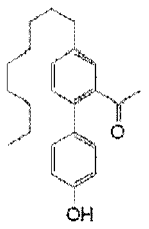
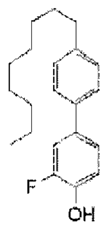
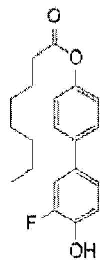
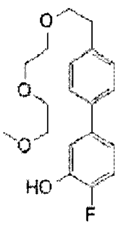
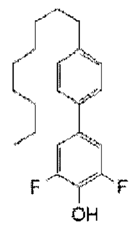

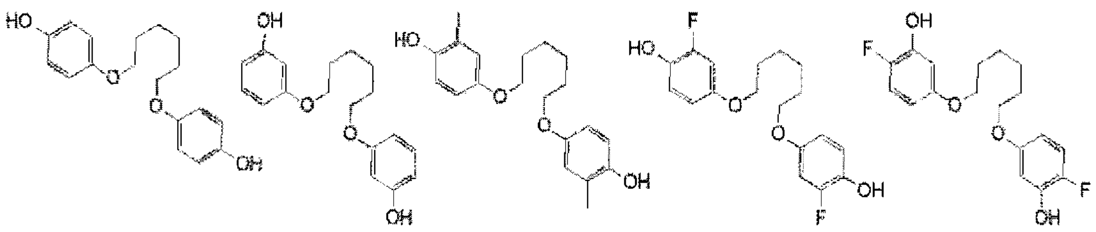

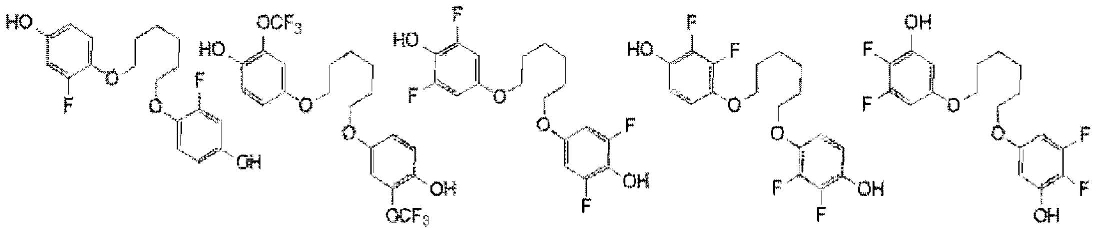

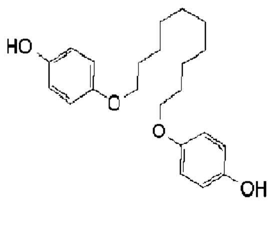
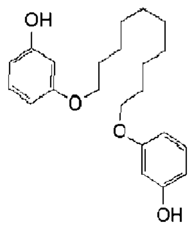
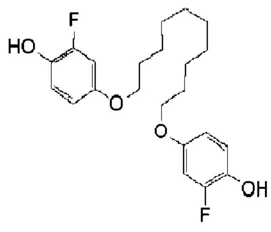
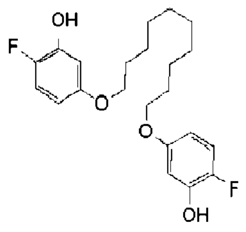

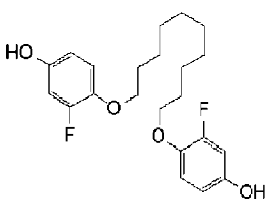
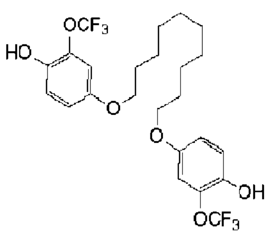
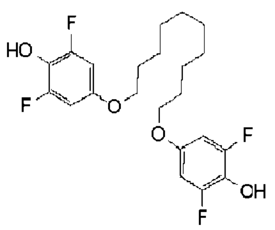
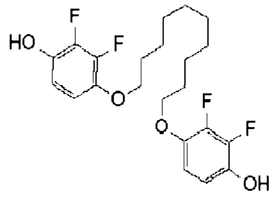

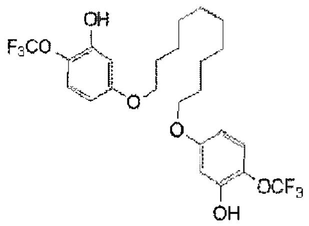
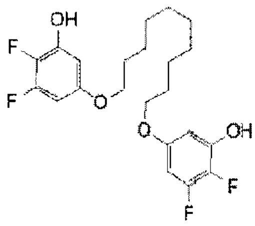
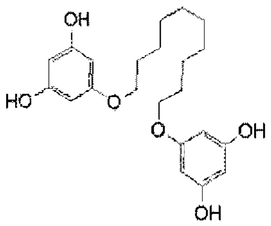
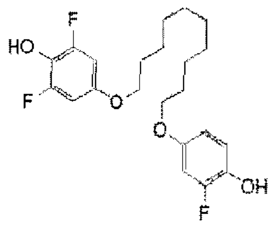

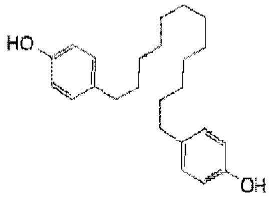
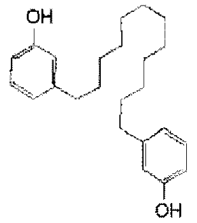
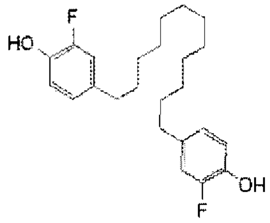
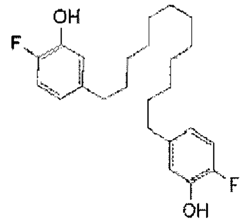

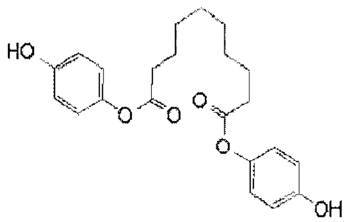
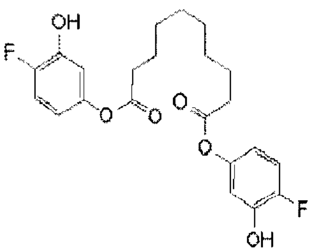
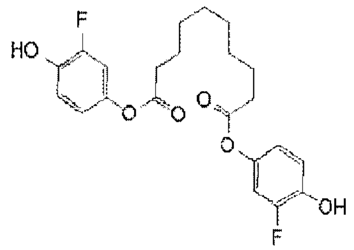

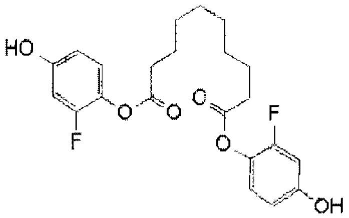
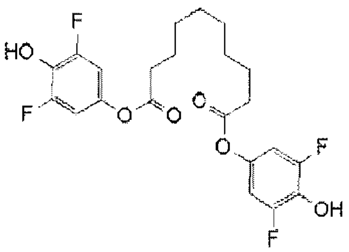
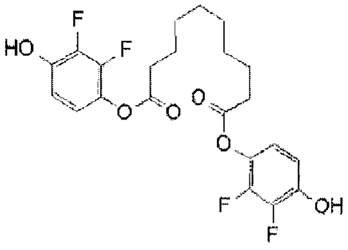

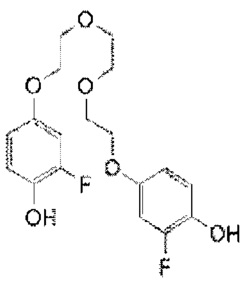
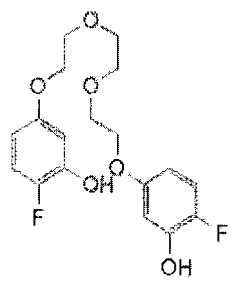
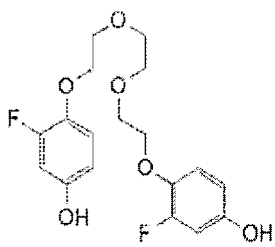
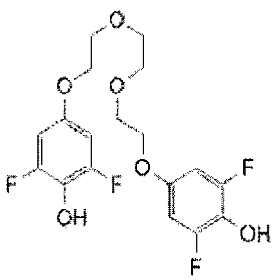

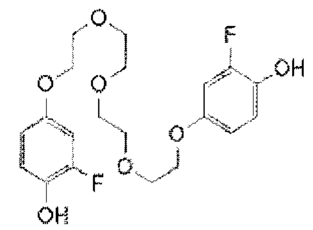
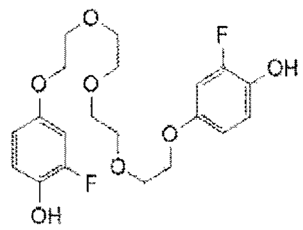
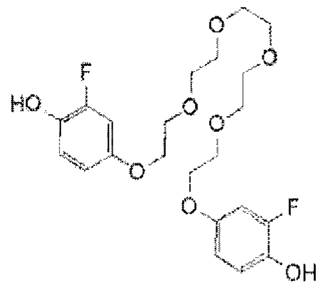

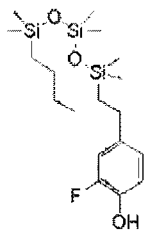
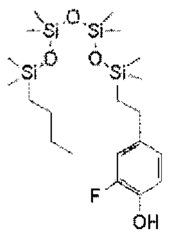
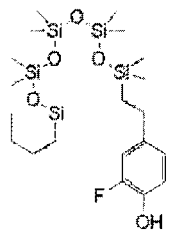
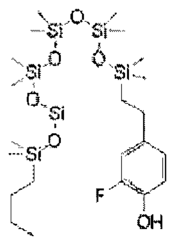
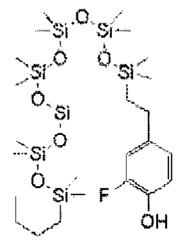
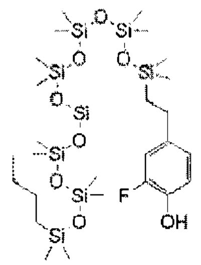

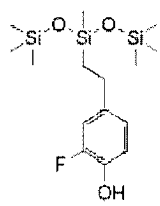
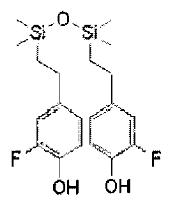
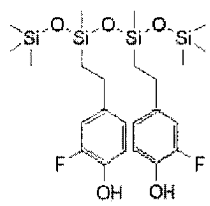
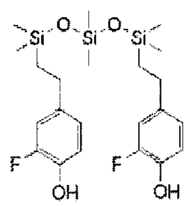

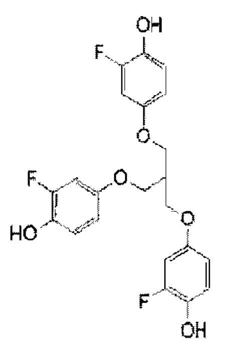
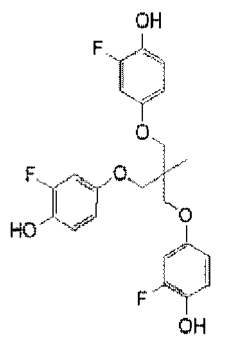
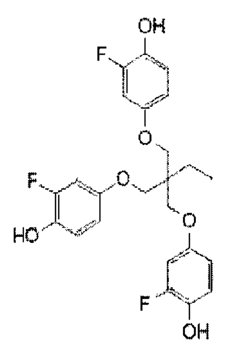
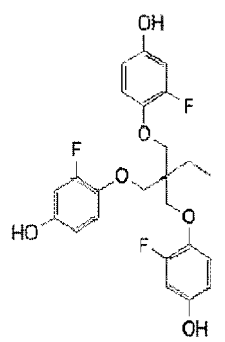

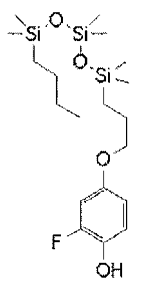
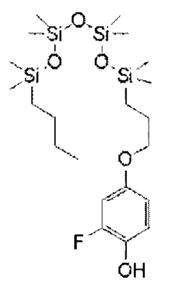
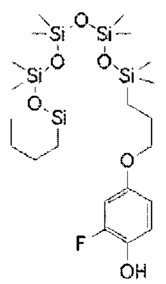
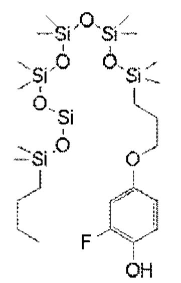
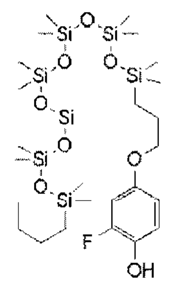
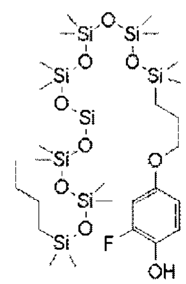

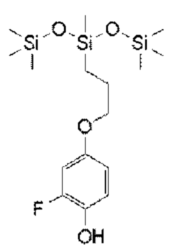
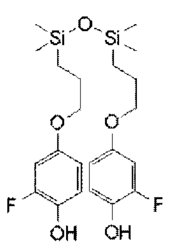
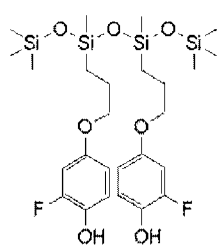
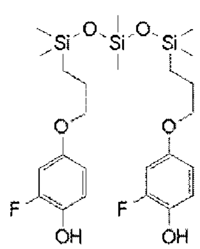

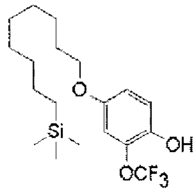 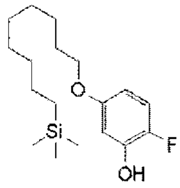 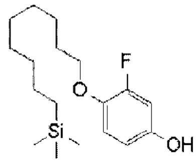 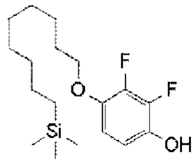 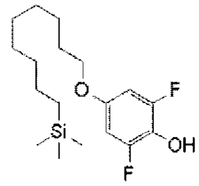

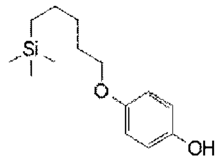 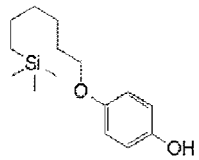 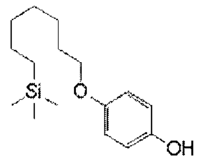 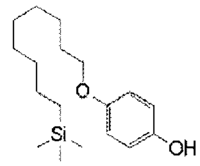 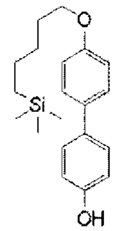

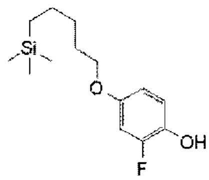 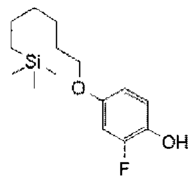 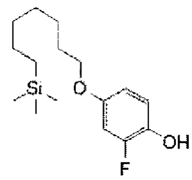 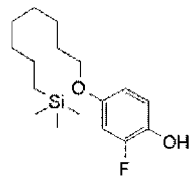 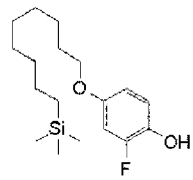

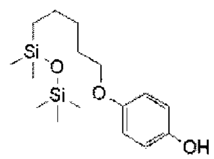 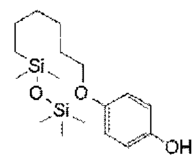 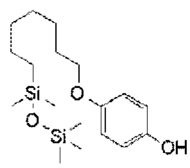 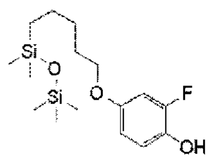 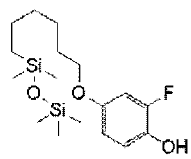

The blending amount of the component (F) is preferably 0.01 to 10 mass% relative to the entire composition, more preferably 0.03 to 5 mass%, and further preferably 0.05 to 1.0 mass%. When the blending amount of the component (F) is 0.01 mass% or more, it contributes to improving the heat dissipation properties of the thermally conductive addition-curable silicone composition. When the amount is 10 mass% or less, the heat dissipation improvement effect is sufficient and economical.

Other Components

The inventive thermally conductive addition-curable silicone composition may contain an organo(poly)siloxane having no reactivity such as methylpolysiloxane in order to regulate strength and viscosity of the composition. For a purpose of improving filling ability of the thermally conductive filler or a purpose of imparting adhesiveness to the composition, a hydrolysable organopolysiloxane, modified silicones, and a hydrolysable organosilane may be further blended. Furthermore, a solvent for regulating the viscosity of the composition may be further blended. To prevent deterioration of the thermally conductive addition-curable silicone composition, a conventionally publicly known oxidation inhibitor, such as 2,6-di-tert-butyl-4-methylphenol, may be further contained as necessary. A dye, a pigment, a flame retardant, a precipitation inhibitor, a thixotropic improver, etc., may be further blended as necessary.

Method for manufacturing thermally conductive addition-curable silicone composition

A method for manufacturing the inventive thermally conductive addition-curable silicone composition is not particularly limited, but includes a step of preparing a thermally conductive addition-curable silicone composition containing the components (A) to (E) described above, and in addition, the component (F) and other components as necessary.

Examples thereof include a method of mixing the components (A) to (E), and as necessary the component (F) and other components by using a mixer, such as, for example, Trimix, Twin Mix, and Planetary Mixer (all are registered trademarks of mixers manufactured by INOUE MFG., INC.), Ultra Mixer (a registered trademark of a mixer manufactured by MIZUHO INDUSTRIAL CO., LTD.), HIVIS MIX (a registered trademark of a mixer manufactured by PRIMIX Corporation), or the like at 25°C for typically 3 minutes to 24 hours, preferably 5 minutes to 12 hours, and particularly preferably 10

minutes to 6 hours. In the mixing, degassing may be performed, and the mixing may be performed while heating within a range of 40 to 170°C.

In the present invention, it is preferable that the components (A) and (B) are mixed in advance at 70°C, and then the components (C), (D), and (E) are mixed at 25°C from the viewpoint of exhibiting good thermal conductivity of the thermally conductive addition-curable silicone composition. When the component (F), which is an optional component, is blended, it is preferable that the components (A) and (B) are mixed in advance, then the component (F) is blended thereto and mixed, and then the components (C), (D), and (E) are is blended thereto and mixed.

The inventive thermally conductive addition-curable silicone composition has a viscosity measured at 25°C of preferably 10 to 1,000 Pa·s, more preferably 20 to 700 Pa·s, and further preferably 40 to 600 Pa·s. When the viscosity is 10 Pa·s or more, shape retention is easy, and operability improves because the thermally conductive filler does not precipitate. When the viscosity is 1,000 Pa·s or less, discharging and applying is easy, and operability improves. The viscosity can be achieved by regulating the blending amount of each of the aforementioned components. The viscosity can be measured at 25°C by using, for example, a Malcom viscosimeter (type PC-1T).

A cured product of the inventive thermally conductive addition-curable silicone composition has typically a thermal conductivity of 0.5 to 100 W/m·K. The thermal conductivity can be derived, for example, from the following equation:

(Thickness of silicone composition [μm]) ÷ (Thermal resistance value [$mm^2$ ·K/W] of silicone composition)

The thermal resistance value can be measured, for example, using NanoFlash (LFA 447, manufactured by NETZSCH) .

Curing conditions for heating and curing the inventive thermally conductive addition-curable silicone composition are not particularly limited, and typically 80 to 200°C, preferably 100 to 180°C, for 15 minutes to 4 hours, preferably 30 minutes to 2 hours.

## EXAMPLE

Hereinafter, the present invention will be described in more detail with reference to Examples and Comparative examples, but is not limited to the examples described below. Structural analysis of the compounds was performed using a Bruker nuclear magnetic resonance (NMR) spectrometer, model Avance III HD NanoBay 400 MHz, and a PerkinElmer Fourier transform infrared (FTIR) spectrometer, model Spectrum One.

The inventive bicyclic acetylene alcohol compounds 1 to 3 were synthesized by the following method.

[Synthesis Example 1]

Synthesis of bicyclic acetylene alcohol compound 1

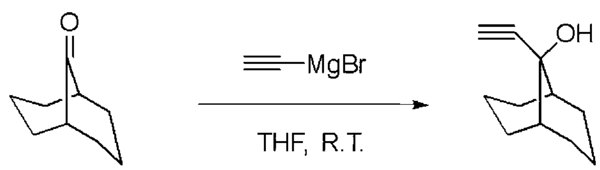


Bicyclic acetylene alcohol compound 1

Under a nitrogen atmosphere, bicyclo[3.3.1]nonan-9-one (0.69 g) was dissolved in THF (5.0 mL) and cooled to 0°C. A THF solution (15.0 mL) of 0.5 M ethynylmagnesium bromide was added carefully thereto dropwise while maintaining the internal temperature below 10°C. The mixture was then stirred at room temperature for 24 hours. Next, the mixture was added with 0.5 N hydrochloric acid aqueous solution (20.0 mL) and stirred at room temperature for 10 minutes. The aqueous layer after the organic layer was separated was extracted three times with ethyl acetate (40 mL), combined with the organic layer, and neutralized and washed with tap water. The organic layer after the neutralization and washing was added with an anhydrous sodium sulfate (40 g) and stirred at room temperature for 3 hours. After filtering off the sodium sulfate, the mixture was concentrated to obtain the light brown solid, a bicyclic acetylene alcohol compound 1 (0.70 g, yield 85%).

$^1$H-NMR (400 MHz in DMSO-d6): δ = 5.24 (1H, s), 3.25 (1H, s), 2.10-1.32 (14H, m) ppm.

[Synthesis Example 2]

Synthesis of bicyclic acetylene alcohol compound 2

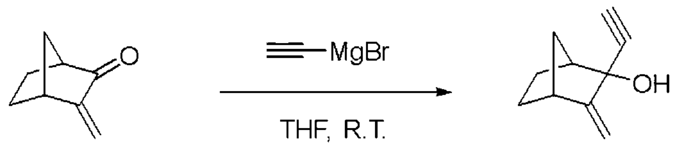


Bicyclic acetylene alcohol compound 2

Under a nitrogen atmosphere, 3-methylene-2-norbornanone (2.44 g) was dissolved in THF (20 mL) and cooled to 0°C. A THF solution (60.0 mL) of 0.5 M ethynylmagnesium bromide was added carefully thereto dropwise while maintaining the internal temperature below 10°C. The mixture was then stirred at room temperature for 24 hours. Next, the mixture was added with 0.5 N hydrochloric acid aqueous solution (80.0 mL) and stirred at room temperature for 10 minutes. The aqueous layer after the organic layer was separated was extracted three times with ethyl acetate (120 mL), combined with the organic layer, and neutralized and washed with tap water. The organic layer after the neutralization and washing was added with an anhydrous sodium sulfate (140g) and stirred at room temperature for 3 hours. After filtering off the sodium sulfate, the mixture was concentrated to obtain an orange-brown transparent liquid, a bicyclic acetylene alcohol compound 2 (2.48 g, yield 84%).

$^1$H-NMR (400 MHz in DMSO-d6): $\delta$ = 5.63 (1H, s), 4.96 (1H, s), 4.85 (1H, s), 3.20 (1H, s), 2.67 (1H, s), 2.31 (1H, s), 1.87-1.27 (8H, m) ppm.

[Synthesis Example 3]

Synthesis of bicyclic acetylene alcohol compound 3

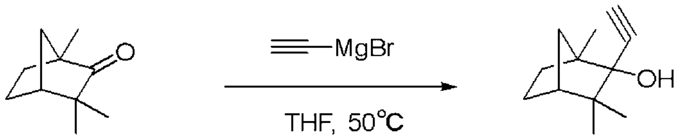


Bicyclic acetylene alcohol compound 3

Under a nitrogen atmosphere, (+)-fenchone (2.28 g) was dissolved in THF (15 mL) and cooled to 0°C. A THF solution (45.0 mL) of 0.5 M ethynylmagnesium bromide was added carefully thereto dropwise while maintaining the internal temperature below 10°C. The mixture was then stirred at 50°C for 7 hours. Next, the mixture was added with 0.5 N hydrochloric acid aqueous solution (60.0 mL) and stirred at room temperature for 10 minutes. The aqueous layer after the organic layer was separated was extracted three times with ethyl acetate (80 mL), combined with the organic layer, and neutralized and washed with tap water. The organic layer after the neutralization and washing was added with an anhydrous sodium sulfate (70 g) and stirred at room temperature for 3 hours. After filtering off the sodium sulfate, the mixture was concentrated to obtain a light orange transparent liquid, a bicyclic acetylene alcohol compound 3 (2.04 g, yield 76%).

$^1$H-NMR (400 MHz in DMSO-d6): $\delta$ = 5.01 (1H, s), 3.26 (1H, s), 2.11 (1H, s), 1.89-0.85 (16H, m) ppm.

The following components to prepare the inventive thermally conductive addition-curable silicone composition were each prepared. Note that the kinematic viscosity indicates the value measured at 25°C using an Ubbelohde-type Ostwald viscometer.

Component (A)

A-1: Dimethylpolysiloxane (SiVi = 0.014 mol/100 g) with both ends blocked with a dimethylvinylsilyl group and having a kinematic viscosity at 25°C of 600 $mm^2$/s.

Component (B)

B-1: Flake-shaped silver powder with an average particle size of 2 μm, a tap density of 2.4 g/mL, and a specific surface area of 0.9 $m^2$/g.
B-2: Spherical silver powder with an average particle size of 3 μm, a tap density of 7.0 g/mL, and a specific surface area of 0.2 $m^2$/g.

Component (C)

C-1: Methyl hydrogen dimethyl polysiloxane represented by the following formula (2).

(kinematic viscosity at 25°C = 100 $mm^2$/s, SiH = 0.0055 mol/g)

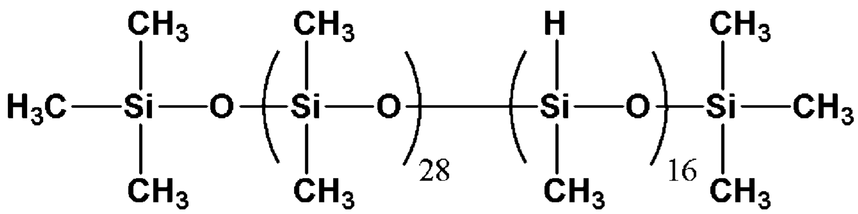

(2)

Component (D)

D-1: Solution of platinum-divinyl tetramethyl disiloxane complex in the same dimethylpolysiloxane as A-1 above (Platinum atom content: 1 wt%).

Component (E)

E-1: Bicyclic acetylene alcohol compound 1 represented by the following formula (3), synthesized in Synthesis Example 1.

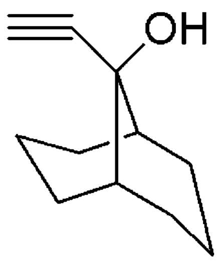

(3)

E-2: Toluene solution of bicyclic acetylene alcohol compound 1 represented by the formula (3) above, synthesized in Synthesis Example 1 (bicyclic acetylene alcohol compound 1 content: 50 wt%).
E-3: Bicyclic acetylene alcohol compound 2 represented by the following formula (4), synthesized in Synthesis Example 2.

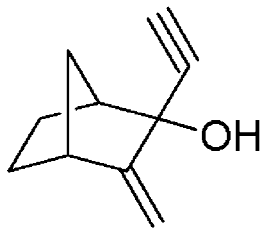

(4)

e-4 (for comparison): acetylene alcohol compound represented by the following formula (5).

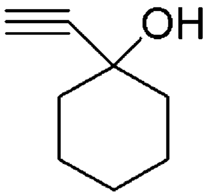

(5)

e-5 (for comparison): acetylene alcohol compound represented by the following formula (6).

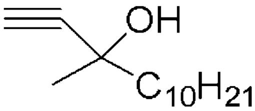

(6)

Component (F)

F-1: Phenol compounds represented by the following formula (7).

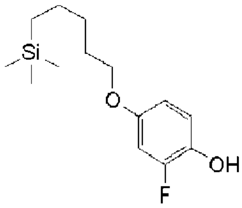

(7)

[Examples 1 to 3 and Comparative Examples 1 to 3]

Preparation of thermally conductive addition-curable silicone composition

The above components (A) to (F) were blended at the blending amounts shown in the following Tables 1 to 2 according to the following method to prepare thermally conductive addition-curable silicone compositions. In Tables, "SiH/SiVi" refers to a ratio of a total number of SiH groups in the component (C) relative to a total number of alkenyl groups in the component (A).

The components (A) and (B) were added to a 0.3-L HIVIS MIX (manufactured by PRIMIX Corporation), and the mixture was mixed at 70°C for 1 hour. This mixture was cooled to 40°C or lower, then added with the components (F), (E), (D), and (C), and mixed for uniformization to prepare the thermally conductive addition-curable silicone composition.

Viscosity, thermal conductivity, and pot life at 25°C of each of the thermally conductive addition-curable silicone compositions obtained by the above method were measured according to the following methods. Tables 1 to 2 show the results.

[Viscosity]

An absolute viscosity of each of the thermally conductive addition-curable silicone compositions was measured at 25°C by using a Malcom viscosimeter (type PC-1T) (with rotor A at 10 rpm, shear rate: 6 [1/s]).

[Thermal Conductivity]

Each of the thermally conductive addition-curable silicone compositions was sandwiched between two silicon plates having a diameter of 12.7 mm, the composition was heated and cured at 125°C for 1 hour while applying a pressure of 0.14 MPa to produce a specimen for measuring thermal resistivity, and a thermal resistivity value of the cured product of the thermally conductive addition-curable silicone composition was measured. Further, the thickness of the specimen was measured with a microgauge, and the thickness of the cured product of the thermally conductive addition-curable silicone composition was calculated as the difference between the thickness of the silicon plates measured in advance and the thickness of the specimen. Thereafter, the thermal conductivity of the cured product of the thermally conductive addition-curable silicone composition was derived by the following formula. (the thickness (μm) of the cured product of a thermally conductive addition-curable silicone composition) / (the thermal resistivity value ($mm^2 \cdot K/W$) of the cured product of a thermally conductive addition-curable silicone composition)

Note that the thermal resistivity was measured by using Nanoflash (LFA447, manufactured by NETZSCH).

[Pot Life at 25°C]

The absolute viscosity of each thermally conductive addition-curable silicone composition was measured at 25°C using a Malcolm viscometer (Type PC-1T) (Rotor A at 10 rpm, shear rate 6 [1/s]). After storing the samples at 25°C for 24 hours, the absolute viscosity was measured again in the same manner, and the difference in the viscosity before and after storage was calculated.

[Table 1]

| | | Example | | |
|---|---|---|---|---|
| | | 1 | 2 | 3 |
| Composition | A-1 (parts by mass) | 26.4 | 26.4 | 26.4 |
| | B-1 (parts by mass) | 37.5 | 37.5 | 37.5 |
| | B-2 (parts by mass) | 338 | 338 | 338 |
| | Mass% of component (B) to the entire composition | 92.6 | 92.6 | 92.6 |
| | C-1 (parts by mass) | 0.81 | 0.81 | 0.81 |
| | D-1 (parts by mass) | 0.69 | 0.69 | 0.69 |
| | E-1 (parts by mass) | 0.24 | | |
| | E-2 (parts by mass) | | 0.48 | |
| | E-3 (parts by mass) | | | 0.22 |
| | F-1 (parts by mass) | 1.9 | 1.9 | 1.9 |
| | SiH/SiVi (ratio in number) | 1.2 | 1.2 | 1.2 |
| Evaluation result | Initial viscosity (Pa·s) | 305 | 330 | 271 |
| | Viscosity after 24 hours at 25°C (Pa·s) | 401 | 403 | 372 |
| | Difference in viscosity ("after 24 hours at 25°C" - "Initial", Pa·s) | 96 | 73 | 101 |
| | Thermal conductivity (W/m·K) | 10 | 12 | 8.4 |

[Table 2]

| | | Comparative example | | |
|---|---|---|---|---|
| | | 1 | 2 | 3 |
| Composition | A-1 (parts by mass) | 26.4 | 26.4 | 26.4 |
| | B-1 (parts by mass) | 37.5 | 37.5 | 37.5 |
| | B-2 (parts by mass) | 338 | 338 | 338 |
| | Mass% of component (B) to the entire composition | 92.6 | 92.6 | 92.6 |
| | C-1 (parts by mass) | 0.81 | 0.81 | 0.81 |
| | D-1 (parts by mass) | 0.69 | 0.46 | 0.69 |
| | e-4 (parts by mass) | 0.18 | 0.18 | |
| | e-5 (parts by mass) | | | 0.30 |
| | F-1 (parts by mass) | 1.9 | 1.9 | 1.9 |
| | SiH/SiVi (ratio in number) | 1.2 | 1.2 | 1.2 |
| Evaluation result | Initial viscosity (Pa·s) | 192 | 245 | 332 |
| | Viscosity after 24 hours at 25°C (Pa·s) | 380 | 431 | 503 |
| | Difference in viscosity ("after 24 hours at 25°C" - "Initial", Pa·s) | 188 | 186 | 171 |
| | Thermal conductivity (W/m·K) | 9.3 | 11 | 10 |

Table 1 results show that the thermally conductive addition-curable silicone compositions of Examples 1 to 3, in which the inventive bicyclic acetylene alcohol compound is blended as an addition-curing reaction-controlling agent, exhibit a small difference between the viscosity after 24 hours of storage at 25°C and the initial viscosity.

On the other hand, Table 2 results show that the thermally conductive addition-curable silicone compositions of Comparative Examples 1 to 3, in which the inventive bicyclic acetylene alcohol compound is not blended as an addition-curing reaction-controlling agent, exhibit a big difference between the viscosity after 24 hours of storage at 25°C and the initial viscosity.

Specifically, it can be seen that the thermally conductive addition-curable silicone compositions containing the inventive bicyclic acetylene alcohol compound as an addition-curing reaction-controlling agent exhibit an extended pot life at 25°C.

The present description includes the following embodiments.

[1]: A bicyclic acetylene alcohol compound, wherein the carbon atom on which the hydroxy group of the compound is substituted is a tertiary carbon atom bonded to an acetylene group, and an $\alpha$-carbon of the compound is a bridgehead carbon atom, or does not have a hydrogen atom on the $\alpha$-carbon, or satisfies both of these conditions.

[2]: The bicyclic acetylene alcohol compound of the above [1], wherein the minimum number of carbon atoms constituting a ring is 7 or fewer.

[3]: An addition-curable silicone composition comprising an effective amount of the bicyclic acetylene alcohol compound of the above [1] or [2] as an addition-curing reaction-controlling agent.

[4]: A thermally conductive addition-curable silicone composition comprising:

(A) organopolysiloxane having at least one aliphatic unsaturated hydrocarbon group per molecule and a kinematic viscosity at 25°C of 60 to 100,000 $mm^2/s$: an amount of constituting 1.5 to 89 mass% relative to the entire composition;

(B) at least one thermally conductive filler selected from the group consisting of a metal, a metal oxide, a metal hydroxide, a metal nitride, a metal carbide, and a carbon allotrope: an amount of 10 to 98 mass% relative to the

entire composition;
(C) organohydrogenpolysiloxane having two or more hydrogen atoms bonded to a silicon atom per molecule: an effective amount sufficient for the composition to form a cured product;
(D) a platinum group metal catalyst: an effective amount; and
(E) the bicyclic acetylene alcohol compound of the above [1] or [2]: an effective amount.

[5]: The thermally conductive addition-curable silicone composition of the above [4], wherein the component (B) is silver powder.
[6]: The thermally conductive addition-curable silicone composition of the above [4] or [5], further comprising (F) a phenol compound represented by the following general formula (1A): an amount of 0.01 to 10 mass% relative to the entire composition,

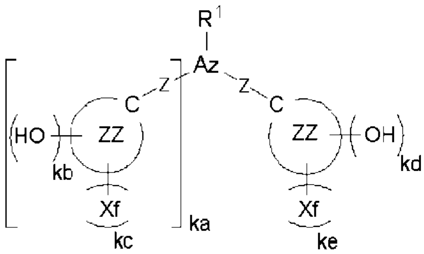
(1A)

wherein $R^1$ represents a hydrogen atom, a halogen atom, a cyano group, or a hydroxy group; Az represents a linear, branched, or cyclic (ka+2)-valent hydrocarbon group having 1 to 20 carbon atoms or a linear, branched, or cyclic (ka+2)-valent fluorinated hydrocarbon group having 1 to 20 carbon atoms, wherein $-CH_2-$ constituting the hydrocarbon group is optionally substituted with -O-, -C(=O)-, or -Si ($R^2R^3$) -; $R^2$ and $R^3$ represent a linear, branched, or cyclic alkyl group having 1 to 6 carbon atoms or a phenyl group, wherein $-CH_2-$ constituting the alkyl group is optionally substituted with -O-, -C(=O)-, or $-Si(CH_3)_2-$; Xf each independently represents a hydrogen atom, a halogen atom, a linear, branched, or cyclic monovalent hydrocarbon group having 1 to 10 carbon atoms and optionally being substituted with a fluorine atom, an alkoxy group having 1 to 10 carbon atoms and optionally being substituted with a fluorine atom, or an electron-withdrawing group; Z represents a single bond or an oxygen atom; rings ZZ each independently represent an aromatic monocyclic or polycyclic ring having 3 to 20 carbon atoms, wherein a carbon atom of the ring ZZ is optionally substituted with a nitrogen atom, an oxygen atom, or a sulfur atom; ka represents an integer of 0 to 2; kb and kd represent 1 or 2; and kc and ke represent an integer of 0 to 2.
[7]: The thermally conductive addition-curable silicone composition of the above [6], wherein the component (F) is a phenol compound represented by the following general formula (1B),

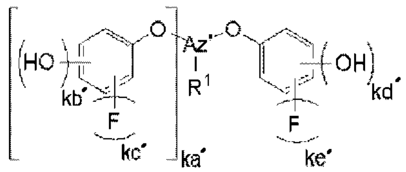
(1B)

wherein Az' represents a linear, branched, or cyclic (ka'+2)-valent hydrocarbon group having 1 to 19 carbon atoms or a linear, branched, or cyclic (ka'+2)-valent fluorinated hydrocarbon group having 1 to 19 carbon atoms, and $-CH_2-$ constituting the hydrocarbon group is optionally substituted with -O-, -C(=O)-, or - Si ($R^2R^3$) -; ka' represents 0 or 1; kb', kc', kd', and ke' represent 1 or 2; and $R^1$, $R^2$, and $R^3$ are same as defined above.

It should be noted that the present invention is not limited to the above-described embodiments. The embodiments are just examples, and any examples that have substantially the same feature and demonstrate the same functions and effects as those in the technical concept disclosed in claims of the present invention are included in the technical scope of the present invention.

## Claims

1. A bicyclic acetylene alcohol compound, wherein the carbon atom on which the hydroxy group of the compound is substituted is a tertiary carbon atom bonded to an acetylene group, and an $\alpha$-carbon of the compound is a bridgehead

carbon atom, or does not have a hydrogen atom on the α-carbon, or satisfies both of these conditions.

2. The bicyclic acetylene alcohol compound according to claim 1, wherein the minimum number of carbon atoms constituting a ring is 7 or fewer.

3. An addition-curable silicone composition comprising an effective amount of the bicyclic acetylene alcohol compound according to claim 1 or 2 as an addition-curing reaction-controlling agent.

4. A thermally conductive addition-curable silicone composition comprising:

(A) organopolysiloxane having at least one aliphatic unsaturated hydrocarbon group per molecule and a kinematic viscosity at 25°C of 60 to 100,000 $mm^2/s$: an amount of constituting 1.5 to 89 mass% relative to the entire composition;
(B) at least one thermally conductive filler selected from the group consisting of a metal, a metal oxide, a metal hydroxide, a metal nitride, a metal carbide, and a carbon allotrope: an amount of 10 to 98 mass% relative to the entire composition;
(C) organohydrogenpolysiloxane having two or more hydrogen atoms bonded to a silicon atom per molecule: an effective amount sufficient for the composition to form a cured product;
(D) a platinum group metal catalyst: an effective amount; and
(E) the bicyclic acetylene alcohol compound according to claim 1 or 2: an effective amount.

5. The thermally conductive addition-curable silicone composition according to claim 4, wherein the component (B) is silver powder.

6. The thermally conductive addition-curable silicone composition according to claim 4, further comprising

(F) a phenol compound represented by the following general formula (1A): an amount of 0.01 to 10 mass% relative to the entire composition,

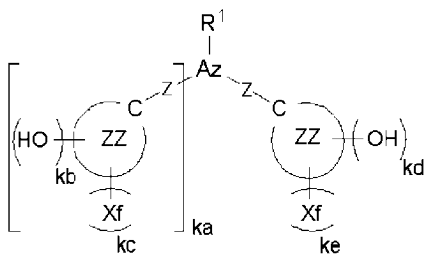

(1A)

wherein $R^1$ represents a hydrogen atom, a halogen atom, a cyano group, or a hydroxy group; Az represents a linear, branched, or cyclic (ka+2)-valent hydrocarbon group having 1 to 20 carbon atoms or a linear, branched, or cyclic (ka+2)-valent fluorinated hydrocarbon group having 1 to 20 carbon atoms, wherein $-CH_2-$ constituting the hydrocarbon group is optionally substituted with -O-, -C(=O)-, or $-Si(R^2R^3)-$; $R^2$ and $R^3$ represent a linear, branched, or cyclic alkyl group having 1 to 6 carbon atoms or a phenyl group, wherein $-CH_2-$ constituting the alkyl group is optionally substituted with -O-, -C(=O)-, or $-Si(CH_3)_2-$; Xf each independently represents a hydrogen atom, a halogen atom, a linear, branched, or cyclic monovalent hydrocarbon group having 1 to 10 carbon atoms and optionally being substituted with a fluorine atom, an alkoxy group having 1 to 10 carbon atoms and optionally being substituted with a fluorine atom, or an electron-withdrawing group; Z represents a single bond or an oxygen atom; rings ZZ each independently represent an aromatic monocyclic or polycyclic ring having 3 to 20 carbon atoms, wherein a carbon atom of the ring ZZ is optionally substituted with a nitrogen atom, an oxygen atom, or a sulfur atom; ka represents an integer of 0 to 2; kb and kd represent 1 or 2; and kc and ke represent an integer of 0 to 2.

7. The thermally conductive addition-curable silicone composition according to claim 6, wherein the component (F) is a phenol compound represented by the following general formula (1B),

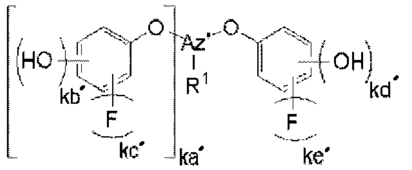 (1B)

wherein Az' represents a linear, branched, or cyclic (ka'+2)-valent hydrocarbon group having 1 to 19 carbon atoms or a linear, branched, or cyclic (ka'+2)-valent fluorinated hydrocarbon group having 1 to 19 carbon atoms, and $-CH_2-$ constituting the hydrocarbon group is optionally substituted with -O-, -C(=O)-, or - Si($R^2R^3$)-; ka' represents 0 or 1; kb', kc', kd', and ke' represent 1 or 2; and $R^1$, $R^2$, and $R^3$ are same as defined above.

## INTERNATIONAL SEARCH REPORT

International application No. **PCT/JP2024/019556**

**A. CLASSIFICATION OF SUBJECT MATTER**

***C07C 35/29***(2006.01)i; ***C07C 35/31***(2006.01)i; ***C07F 7/12***(2006.01)i; ***C08K 3/00***(2018.01)i; ***C08K 3/08***(2006.01)i; ***C08K 5/05***(2006.01)i; ***C08K 5/13***(2006.01)i; ***C08L 83/05***(2006.01)i; ***C08L 83/07***(2006.01)i
FI: C07C35/29; C07C35/31 CSP; C07F7/12 R; C08K3/00; C08K3/08; C08K5/05; C08K5/13; C08L83/05; C08L83/07

According to International Patent Classification (IPC) or to both national classification and IPC

**B. FIELDS SEARCHED**

Minimum documentation searched (classification system followed by classification symbols)

C07C35/29; C07C35/31; C07F7/12; C08K3/00; C08K3/08; C08K5/05; C08K5/13; C08L83/05; C08L83/07

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | JP 2020-531545 A (ENANTA PHARMACEUTICALS, INC.) 05 November 2020 (2020-11-05) particularly, example 339, step 339a | 1-2 |
| Y | | 1-7 |
| X | JP 2016-504300 A (BAYER CROPSCIENCE AG) 12 February 2016 (2016-02-12) particularly, compound no. 1-61, 1-107, 27-30 | 1-2 |
| Y | | 1-7 |
| X | JP 2010-526083 A (ABBOTT LABORATORIES) 29 July 2010 (2010-07-29) particularly, examples 42, 44 | 1-2 |
| Y | | 1-7 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 August 2024** | **13 August 2024** |
| Name and mailing address of the ISA/JP<br>**Japan Patent Office (ISA/JP)**<br>**3-4-3 Kasumigaseki, Chiyoda-ku, Tokyo 100-8915**<br>**Japan** | Authorized officer<br><br>Telephone No. |

Form PCT/ISA/210 (second sheet) (July 2022)

INTERNATIONAL SEARCH REPORT

International application No. PCT/JP2024/019556

C. DOCUMENTS CONSIDERED TO BE RELEVANT

| Category* | Citation of document, with indication, where appropriate, of the relevant passages | Relevant to claim No. |
|---|---|---|
| X<br>Y | JP 58-109434 A (SOCIETE ANONYME ROURE BERTRAND DUPONT) 29 June 1983 (1983-06-29)<br>particularly, example 3 | 1-2<br>1-7 |
| X<br>Y | CAS REGISTRY No. 2184791-97-5, 2166463-17-6, 2166358-99-0. DATABASE REGISTRY. [online]. 2018 (date of receipt), [retrieved on: 18 July 2024], Retrieved from: STN<br>particularly, chemical structure, name | 1-2<br>1-7 |
| X<br>Y | JONES, K. E. et al. Rearrangements of the Chrysanthenol Core: Application to a Formal Synthesis of Xishacorene B. Journal of the American Chemical Society. 2021, vol. 143, no. 48, pp. 20482-20490, DOI: 10.1021/jacs.1c10804<br>particularly, in the case of Entries 4-5 of compounds 10, 12, compounds 10P, 16 | 1-2<br>1-7 |
| X<br>Y | RUEDI, G. et al. An Unusual Domino Retro-Ene-Conia Reaction: Regio- and Stereoselective One-Carbon Ring Expansion of Fenchol Derivatives. Helvetica Chimica Acta. 2004, vol. 87, no. 8, pp. 1990-2021, DOI: 10.1002/hlca.20049018<br>particularly, compound 6, isotope-labeled compounds | 1-2<br>1-7 |
| X<br>Y | SCHOTTENBERGER, H. et al. Mixed-membered-bisfulvalene dimetal-complexes via metallocene-substituted norbornenyl alcohols. Journal of Organometallic Chemistry. 1997, vol. 541, no. 1-2, pp. 249-260, DOI: 10.1016/S0022-328X(97)00068-5<br>particularly, compounds 10a-10b | 1-2<br>1-7 |
| X<br>Y | GOSSELIN, P. et al. Synthesis of earthy-mouldy smelling compounds-II ethyl α- and β-fenchols. Tetrahedron Letters. 1990, vol. 31, no. 22, pp. 3151-3154, DOI: 10.1016/S0040-4039(00)94718-X<br>particularly, compounds 4α, 4β, 4αβ | 1-2<br>1-7 |
| X<br>Y | MARTIN, J. et al. The stereoselective synthesis of meso-trans-1,3-dimethylcyclohexane-2-acetic-1,3-dicarboxylic acid. Chemical Communications. 1965, no. 24, pp. 633-634, DOI: 10.1039/C19650000633<br>particularly, compound IV (when R is H) | 1-2<br>1-7 |
| X<br>Y | GORLICH, B. et al. Umsetzungen von Norcampher und -derivaten mit Acetylen. Athinierung bicyclischer Ketone. Chemische Berichte. 1958, vol. 91, no. 11, pp. 2388-2394<br>particularly, compounds III, IV | 1-2<br>1-7 |
| X<br>Y | LIAO, K. et al. Highly Enantioselective CuAAC of Functional Tertiary Alcohols Featuring an Ethynyl Group and Their Kinetic Resolution. Angewandte Chemie International Edition. 2021, vol. 60, no. 15, pp. 8488-8493, DOI: 10.1002/anie.202016286<br>particularly, compounds 4a-4f, 4h-4j, 6a-6c | 1-2<br>1-7 |
| X<br>Y | FEHR, C. et al. Copper-Catalyzed Cycloisomerizations of 5-En-1-yn-3-ols. Organic Letters. 2006, vol. 8, no. 9, pp. 1839-1841, DOI: 10.1021/ol0603864<br>particularly, compounds 1ab,1a | 1-2<br>1-7 |
| X<br>Y | KUWATANI, Y. et al. Nickel-Catalyzed Dimerization of [5]Cumulenes (Hexapentaenes). Bulletin of the Chemical Society of Japan. 2005, vol. 78, no. 12, pp. 2188-2208, DOI: 10.1246/bcsj.78.2188<br>particularly, compound 25b | 1-2<br>1-7 |

Form PCT/ISA/210 (second sheet) (July 2022)

**INTERNATIONAL SEARCH REPORT**

International application No.
**PCT/JP2024/019556**

**C. DOCUMENTS CONSIDERED TO BE RELEVANT**

| Category* | Citation of document, with indication, where appropriate, of the relevant passages | Relevant to claim No. |
|---|---|---|
| X | SNIDER, B. B. et al. Manganese(III)-based oxidative fragmentation-cyclization reactions of unsaturated cyclobutanols. The Journal of Organic Chemistry. 1993, vol. 58, no. 25, pp. 7228-7237, DOI: 10.1021/jo00077a054 particularly, compound 49 | 1-2 |
| Y | | 1-7 |
| Y | JP 2021-185215 A (SHIN-ETSU CHEMICAL CO., LTD.) 09 December 2021 (2021-12-09) claims, paragraphs [0043], [0046] | 1-7 |
| Y | JP 2021-193168 A (SHIN-ETSU CHEMICAL CO., LTD.) 23 December 2021 (2021-12-23) claims, paragraphs [0031], [0034] | 1-7 |
| Y | WO 2022/107511 A1 (SHIN-ETSU CHEMICAL CO., LTD.) 27 May 2022 (2022-05-27) claims, paragraphs [0057]-[0070] | 1-7 |

Form PCT/ISA/210 (second sheet) (July 2022)

**INTERNATIONAL SEARCH REPORT**
**Information on patent family members**

International application No.
**PCT/JP2024/019556**

| Patent document cited in search report | Publication date (day/month/year) | Patent family member(s) | Publication date (day/month/year) |
|---|---|---|---|
| JP 2020-531545 A | 05 November 2020 | US 2019/0060258 A1<br>example 339, step 339a<br>WO 2019/046271 A1<br>EP 3675637 A1<br>CN 111263586 A<br>KR 10-2020-0083970 A | |
| JP 2016-504300 A | 12 February 2016 | US 2015/0315146 A1<br>especially compounds no.I.1-61, I.1-107, I.27-30<br>WO 2014/086723 A1<br>EP 2928297 A1<br>CN 104955327 A | |
| JP 2010-526083 A | 29 July 2010 | US 2008/0293713 A1<br>examples 42, 44<br>WO 2008/134690 A1<br>EP 2142552 A1<br>KR 10-2010-0019470 A<br>CN 101720327 A | |
| JP 58-109434 A | 29 June 1983 | EP 74543 A1<br>example 3 | |
| JP 2021-185215 A | 09 December 2021 | (Family: none) | |
| JP 2021-193168 A | 23 December 2021 | (Family: none) | |
| WO 2022/107511 A1 | 27 May 2022 | US 2023/0332008 A1<br>claims, paragraphs [0059]-[0060]<br>EP 4249461 A1<br>KR 10-2023-0076850 A<br>CN 114516787 A | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H11236508 A
- JP 2014024986 A
- JP H04046962 A


### Non-patent literature cited in the description


- *J. Org. Chem.*, 1977, vol. 42, 3403